# EUROPÄISCHE PATENTSCHRIFT

(11) **EP 1 124 541 B1**
(45) Veröffentlichungstag und Bekanntmachung des Hinweises auf die Patenterteilung: **26.11.2003**
(21) Anmeldenummer: 99948859.6
(22) Anmeldetag: 24.09.1999
(51) Int. Cl.: A61K 9/28, A61K 9/20, A61K 47/34, C08F 283/06, C08F 218/08

(54) **VERWENDUNG VON WASSERLÖSLICHEN ODER WASSERDISPERGIERBAREN POLYETHER-HALTIGEN PFROPFPOLYMERISATEN ALS ÜBERZUGSMITTEL, BINDEMITTEL UND/ODER FILMBILDENDER HILFSSTOFF IN PHARMAZEUTISCHEN DARREICHUNGSFORMEN**
APPLICATION OF WATER-SOLUBLE OR WATER-DISPERSIBLE GRAFT POLYMERIZATES WHICH CONTAIN POLY-ETHER AND WHICH ARE USED AS A COATING AGENT, A BINDING AGENT AND/OR AS A FILM-FORMING AUXILIARY AGENT IN PHARMACEUTICAL FORMS OF ADMINISTRATION
UTILISATION DE POLYMERISATS GREFFES HYDROMISCIBLES OU HYDRODISPERSIBLES CONTENANT DES POLYETHERS COMME AGENT DE RECOUVREMENT, LIANT ET/OU SUBSTANCE AUXILIAIRE FILMOGENE DANS DES FORMES D'ADMINISTRATION PHARMACEUTIQUES

(30) Priorität: 30.09.1998 DE 19844903; 11.02.1999 DE 19905906; 08.07.1999 DE 19931667
(43) Veröffentlichungstag der Anmeldung: 22.08.2001
(73) Patentinhaber: BASF AKTIENGESELLSCHAFT, 67056 Ludwigshafen (DE)
(72) Erfinder: GOTSCHE, Michael, D-52066 Aachen (DE); KOLTER, Karl, D-67117 Limburgerhof (DE); SANNER, Axel, D-67227 Frankenthal (DE); ANGEL, Maximilian, D-67105 Schifferstadt (DE); LEINENBACH, Alfred, D-67161 Gönnheim (DE)
(86) Internationale Anmeldenummer: EP9907123
(87) Internationale Veröffentlichungsnummer: WO00018375

(56) Entgegenhaltungen:
- EP-A- 0 524 786
- EP-A- 0 797 987
- US-A- 4 224 427
- US-A- 4 548 990
- US-A- 4 873 086

## Beschreibung

Die Erfindung betrifft die Verwendung von wasserlöslichen oder wasserdispergierbaren Polyether-haltigen Pfropfpolymerisaten als Überzugsmittel, Bindemittel und/oder filmbildender Hilfsstoff in pharmazeutischen Darreichungsformen.

Feste pharmazeutische Darreichungsformen wie Tabletten, Kapseln, Pellets, Granulate, Kristalle etc. werden aus sehr unterschiedlichen Gründen gecoatet, d.h. mit einem Filmüberzug versehen. So kann beispielsweise ein schlechter Geruch oder Geschmack maskiert sowie die Schluckbarkeit verbessert werden. Die Stabilität des Wirkstoffes kann durch das Coating erhöht werden, indem weniger Wasserdampf und Sauerstoff an das Tabletteninnere gelangt. Die Darreichungsformen sehen besser aus und können durch die Einarbeitung von Farbstoffen besser unterschieden werden. Darüber hinaus läßt sich insbesondere die Freisetzungsgeschwindigkeit des Wirkstoffes durch den Filmüberzug einstellen.

Generell unterscheidet man Instant-Release-Formen und Retard- bzw. Slow-Release-Formen.

Bei Instant-Release-Formen sollen der Zerfall der Tablette und die Freisetzung des Wirkstoffs aus der Darreichungsform nach Möglichkeit nicht durch das Coating beeinflußt werden, deshalb muß sich der Filmüberzug schnell im Magensaft auflösen. Daneben muß er über gute Filmeigenschaften verfügen. Die Zugfestigkeit und die Reißdehnung sollten hoch sein, damit der Filmüberzug mechanischen Einwirkungen standhält, wie sie bei der pharmazeutischen Verarbeitung - insbesondere der Konfektionierung - und auch während des Versandes bzw. der Lagerung auftreten.

Ein häufig eingesetztes Produkt für das Coaten von Instant-Release-Tabletten ist Hydroxypropylmethylcellulose (HPMC). Hydroxypropylmethylcellulose weist in wäßriger Lösung bei zunehmender Konzentration einen steilen Viskositätsanstieg auf. Ein ähnliches Verhalten zeigt auch Hydroxypropylcellulose (HPC).

Da die Filmbildnerlösung beim Coaten von Tabletten fein zerstäubt werden muß und die gebildeten Tröpfchen die Oberfläche der Tabletten gut benetzen und auch gut spreiten müssen, darf die Viskosität eine gewisse Grenze (zwischen 150 und 250 mPas), die abhängig ist von der Art der Sprühdüse und des Gerätes, nicht überschreiten. Deshalb können im Falle von HPMC nur verhältnismäßig niedrige Filmbildnerkonzentrationen eingesetzt werden.

Als Empfehlung für die Konzentration von Pharmacoat® 606 (Fa. Shin-etsu) werden in der Literatur 5 bis 7 Gew.-% angegeben (Pharmaceutical Coating Technology, edited by Graham Cole, Taylor and Francis Ltd. 1995 und Technische Merkblätter der Hersteller). Diese geringe Sprühkonzentrationen bedingen relativ lange Verarbeitungszeiten und damit hohe Kosten.

Darüber hinaus zeigt Hydroxypropylmethylcellulose weitere Nachteile u.a. im Benetzungsverhalten, in der Adhesivität auf der Tablettenoberfläche, im Pigmentbindevermögen, in den mechanischen Eigenschaften der Filme, in der Hygroskopizität sowie in der Permeabilität gegenüber Wasserdampf und Sauerstoff, in der Auflösungsgeschwindigkeit und in der Zerfallszeitdifferenz zwischen Filmtabletten und Kern.

Die geringe Elastizität der Filme aus Hydroxypropylmethylcellulose führt häufig dazu, daß die Filmtabletten bei feuchter Lagerung infolge der Quellung des Kerns aufreißen. Auch der Einsatz von Weichmachern ergibt keine nennenswerten Verbesserungen dieses Problems. Er kann vielmehr zu klebrigen Filmen und durch Migration zu Veränderungen der Tabletteneigenschaften führen.

Orale Arzneiformen mit einer Arzneistofffreigabe über einen längeren Zeitraum mit dem Ziel der Wirkungsverlängerung der aktiven Komponente (allgemein Retardarzneiformen) gewinnen zunehmend an Bedeutung. Mit ihr sind eine verbesserte Patientencompliance durch eine reduzierte Einnahmefrequenz, eine Verringerung von Nebenwirkungen durch Vermeidung von Plasmaspitzen, gleichmäßigere Blutspiegel des Arzneistoffs sowie die Vermeidung von lokalen Irritationen vorteilhaft verbunden. Neben der Formulierung von arzneistoffhaltigen Kernen, die mit einem wasserunlöslichen aber semipermeablen bzw. porenhaltigen Film überzogen wurden, durch die der Arzneistoff diffundiert, kann die Steuerung und Verlängerung der Freisetzung durch die Einbettung des Arzneistoffes in Matrices erreicht werden. Weiterhin ist der Einsatz von Ionenaustauscherharzen und therapeutischen Systemen (z.B. OROS) möglich.

Besonders die Einbettung des Arzneistoffs in Hydrokolloidmatrices bietet die Vorteile einer einfachen und preiswerten Herstellung und einer hohen Arzneimittelsicherheit, da Dose Dumping Effekte nicht auftreten können. Die hierfür in der Regel eingesetzten Hilfsstoffe wie Hydroxypropylmethylcellulose (HPMC), Hydroxypropylcellulose, Alginsäure bzw. Alginate sowie Xanthan besitzen Anwendungsnachteile. Hier sind zu nennen: Mangelnde Fließeigenschaften, die eine Direkttablettierung erschweren, eine Abhängigkeit der Arzneistofffreigabe von der Osmolarität (Salzgehalt) und vom pH-Wert des Freisetzungsmediums. Dies gilt ebenso für HPMC wie für Hydroxypropylcellulose. Xanthan und Alginate. Die Verwendung von Xanthan führt ferner zu Tabletten mit geringer Härte, die Direkttablettierung von Alginaten resultiert in Preßlingen mit nur noch geringen retardierenden Eigenschaften (max. 8 h). Die natürlichen Quellstoffe (z.B. Alginate) besitzen insgesamt eine starke Chargenvariabilität.

Überraschend wurde gefunden, daß die im folgenden beschriebenen Polymerisate diese Nachteile nicht besitzen und für die Verwendung in oralen pharmazeutischen Zubereitungen als Matrix für die Wirkstofffreisetzung vorteilhaft sind.

Bindemittel werden in pharmazeutischen Darreichungsformen eingesetzt, um die Verarbeitbarkeit und die mechanische Festigkeit zu erhöhen. Sie werden üblicherweise in Tabletten, Granulaten und Pellets eingesetzt und führen zu verbesserter Fließfähigkeit, höherer Bruchfestigkeit und geringerer Friabilität.

Die derzeit verwendeten Bindemittel wie Maltodextrin oder Polyvinylpyrrolidone führen häufig zu nicht zufriedenstellenden Bruchfestigkeiten und Friabilitäten. Andere Bindemittel wie Stärkekleister und Hydroxypropylmethylcellulose (HPMC) lassen sich aufgrund ihrer hohen Viskosität nur niedrigkonzentriert einsetzen.

Weiterhin werden filmbildende Hilfsstoffe in Lösungen und Sprays eingesetzt, die auf der Haut oder Schleimhaut aufgebracht oder auch dem Körper systemisch zugeführt werden. Beispiele hierfür sind Zubereitungen für die Wundbehandlung, Sprayverbände aber auch Zubereitungen zur Applikation auf intakter Haut bzw. Schleimhaut. Dabei wird die Haut durch einen Film geschützt und die Wirkstoffe können in bzw. durch die Haut dringen.

Bei transdermalen therapeutischen Systemen und bei Wundpflastern ist ebenfalls wie bei den oben genannten Darreichungsformen eine hohe Flexibilität erforderlich, die die derzeit zur Verfügung stehenden Produkte nicht aufweisen. Der Einsatz von möglichen Weichmachern zur Erreichung der notwendigen Flexibilität ist aus toxikologischen und pharmakologischen Gründen nicht erwünscht.

Der vorliegenden Erfindung lag die Aufgabe zugrunde, wasserlösliche oder wasserdispergierbare Polymere als Überzugsmittel, Bindemittel und/oder filmbildende Hilfsstoffe in pharmazeutischen Darreichungsformen bereitzustellen, die die oben genannten Nachteile nicht aufweisen.

Die Aufgabe wurde erfindungsgemäß gelöst durch Verwendung von Pfropfpolymerisaten, insbesondere von solchen Polymerisaten, die wasserlöslich oder wasserdispergierbar sind und die erhältlich sind durch Polymerisation von
a) mindestens einem Vinylester von aliphatischen C₁-C₂₄-Carbonsäuren, in Gegenwart von
b) Polyethern der allgemeinen Formel I, 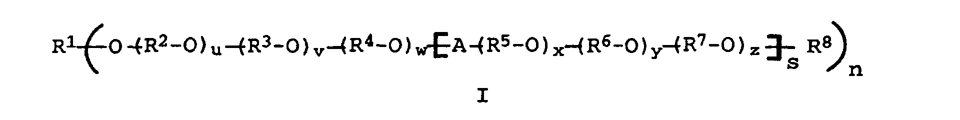
in der die Variablen unabhängig voneinander folgende Bedeutung haben:
- R¹: Wasserstoff, C₁-C₂₄-Alkyl, R⁹-C(=O)-, R⁹-NH-C(=O)-, Polyalkoholrest;
- R⁸: Wasserstoff, C₁-C₂₄-Alkyl, R⁹-C(=O)-, R⁹-NH-C(=O)-;
- R² bis R⁷: -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
- R⁹: C₁-C₂₄-Alkyl;
- R¹⁰: Wasserstoff, C₁-C₂₄-Alkyl, R⁹-C(=O)-;
- A: -C(=O)-O-, -C(=O)-B-C(=O)-O-,
-C(=O)-NH-B-NH-C(=O)-O-;
- B: -(CH₂)ₜ-, Arylen, gegebenenfalls substituiert;
- n: 1 bis 8;
- s: 0 bis 500;
- t: 1 bis 12;
- u: 1 bis 5000;
- v: 0 bis 5000;
- w: 0 bis 5000;
- x: 1 bis 5000;
- y: 0 bis 5000;
- z: 0 bis 5000, dadurch gekennzeichnet, dass das Molekulargewicht der Polyether im Bereich von 300 bis 100 000 liegt,
als Überzugsmittel, Bindemittel und/oder filmbildender Hilfsstoff in pharmazeutischen Darreichungsformen.

Polyalkylenoxid-haltige Pfropfpolymerisate, u.a. auch als Hilfsmittel in pharmazeutischen Zubereitungen sind bereits bekannt.

So beschreibt DE-A-23 63 853 die Verwendung von teilverseiften Pfropfcopolymerisaten von Vinylacetat auf Polyethylenglykol zur Herstellung von selbsttragenden Packungen oder Kapseln für Medikamente. Diese aus den Pfropfpolymerisaten hergestellten Kapseln sollen als Alternative zu den bekannten Hartgelatinekapseln, wie sie beispielsweise beschrieben sind in Pharmazie in unserer Zeit, 23(4), 226-229 (1994), eingesetzt werden. Für die Verwendung der Pfropfpolymerisate als Überzugs- bzw. Bindemittel für pharmazeutische Darreichungsformen finden sich in dieser Schrift keine Hinweise.

DE 1 077 430, DE 1 094 457 und DE 1 081 229 beschreiben Verfahren zur Herstellung von Pfropfpolymerisaten von Polyvinylestern und deren Verwendung als wasserlösliche Verpackungsfolien sowie als Hilfsmittel in der Kosmetik.

DE 43 36 493 beschreibt wasserlösliche oxyalkylengruppenhaltige Polyvinylalkoholharzzusammensetzungen und deren Verwendung beispielsweise als Verpackungsmaterialien.

US 4,548,990 offenbart eine wirkstoffübertragende Carrier-Verbindung mit kontrollierter Freisetzung, die ein quellbares, vernetztes Copolymer umfasst, enthaltend 50 bis 99 Gew.-% wasserunlösliche Monomere.

US 4,224,427 beschreibt ein Verfahren zur Herstellung von einheitlich sphärischen Polymerteilchen zur Suspensionspolymerisation von 30 bis 95 % eines wasserlöslichen monoolefinischen Monomers, 0 bis 70 % eines wasserunlöslichen Monomers und 5 bis 70 % eines vernetzenden Makromonomers. Die beanspruchten Polymerisate sind wasserunlösliche, wasserquellbare Hydrogele.

US 4,873,086 offenbart ein Verfahren zur Herstellung einer Verbindung mit kontrollierter Freisetzung. Bei diesem Verfahren werden zur Herstellung der Polymerisate vernetzende polyethergruppenhaltige Makromonomere eingesetzt. Die Polymerisate sind ebenfalls wasserunlösliche, wasserquellbare Hydrogele.

EP 797 987 umfasst eine Zubereitung, die durch Nassgranulierung einer wässrigen Suspension hergestellt wird und einen medizinischen Inhaltsstoff, ein Polymer und einen Weichmacher beinhaltet.

Bei den erfindungsgemäß verwendeten Polymerisaten handelt es sich um sogenannte Pfropfpolymerisate, bei denen als Pfropfgrundlage b) generell Polyether der allgemeinen Formel I, ausgewählt aus der Gruppe, bestehend aus Polyalkylenoxiden auf Basis von Ethylenoxid, Propylenoxid und Butylenoxid sowie Polyglycerin verwendet werden. Je nach Art der Monomerbausteine ergeben sich Polymere mit folgenden Struktureinheiten.
-(CH₂)₂-O-, -(CH₂)₃-O-, -(CH₂)₄-O-, -CH₂-CH(CH₃)-O-,
-CH₂-CH(CH₂-CH₃)-O-, -CH₂-CHOR⁷-CH₂-O-;

Dabei kann es sich sowohl um Homopolymere als auch um Copolymere handeln, wobei die Copolymere statistisch verteilt sein können oder als sogenannte Blockpolymere vorliegen.

Je nach Pfropfungsgrad sind unter den erfindungsgemäß verwendeten Polymerisaten sowohl reine Pfropfpolymerisate als auch Mischungen der o.g. Pfropfpolymerisate mit ungepfropften Polyethern der Formel I und Homo- oder Copolymerisaten der Monomeren a) und gegebenenfalls weiteren Monomeren c) zu verstehen.

Die endständigen primären Hydroxylgruppen der, auf Basis von Alkylenoxiden oder Glycerin hergestellten Polyether sowie außerdem die sekundären OH-Gruppen von Polyglycerin können sowohl in ungeschützter Form frei vorliegen als auch mit Alkoholen einer Kettenlänge C₁-C₂₄ bzw. mit Carbonsäuren einer Kettenlänge C₁-C₂₄ verethert bzw. verestert werden.

Als Alkylreste für R¹ und R⁸ bis R¹⁰ seien verzweigte oder unverzweigte C₁-C₂₄-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Vertreter der oben genannten Alkylreste seien verzweigte oder unverzweigte C₁-C₁₂-, besonders bevorzugt C₁-C₆-Alkylketten genannt.

Das Molekulargewicht der Polyether liegt im Bereich kleiner 500000 (nach Zahlenmittel), bevorzugt im Bereich von 300 bis 100000, besonders bevorzugt im Bereich von 500 bis 20000, ganz besonders bevorzugt im Bereich von 800 bis 15000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 Mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 Mol.-%, der Propylenoxidanteil 1 bis 60 Mol.-% und der Anteil an Butylenoxid in den Copolymerisaten 1 bis 30 Mol.-%. Neben geradkettigen können auch verzweigte Homo- oder Copolymerisate als Pfropfgrundlage verwendet werden.

Verzweigte Polymerisate können hergestellt werden, indem man beispielsweise an niedrigmolekularen Polyalkoholresten (= R¹ in der allgemeinen Formel I, wie z.B. Pentaerythrit, Glycerin oder an Zuckern bzw. Zuckeralkoholen wie Saccharose, D-Sorbit und D-Mannit) Ethylenoxid und gegebenenfalls noch Propylenoxid und/oder Butylenoxide oder Polyglycerin anlagert.

Dabei können Polymerisate gebildet werden, bei denen mindestens eine, bevorzugt eine bis acht, besonders bevorzugt eine bis fünf der in den Polyalkoholen vorhandenen Hydroxylgruppen in Form einer Etherbindung mit dem folgenden Polyetherrest P, gemäß Formel I 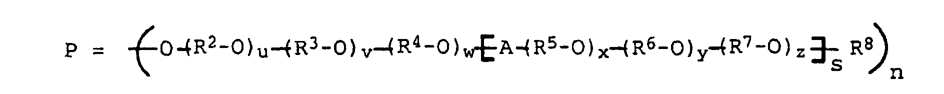 n = 1 bis 8
verknüpft sein können.

Die Alkylenoxid-Einheiten können im Polymerisat statistisch verteilt sein oder in Form von Blöcken vorliegen.

Es ist aber auch möglich, Polyester von Polyalkylenoxiden und aliphatischen C₁-C₁₂-, bevorzugt C₁-C₆-Dicarbonsäuren oder aromatischen Dicarbonsäuren, z.B. Oxalsäure, Bernsteinsäure, Adipinsäure oder Terephthalsäure mit Molmassen von 1500 bis 25000, beschrieben in EP-A-0 743 962, als Pfropfgrundlage zu verwenden.

Es ist weiterhin möglich, durch Phosgenierung hergestellte Polycarbonate von Polyalkylenoxiden oder auch Polyurethane von Polyalkylenoxiden und aliphatischen C₁-C₁₂-, bevorzugt C₁-C₆-Diisocyanaten oder aromatischen Diisocyanaten, z.B. Hexamethylendiisocyanat oder Phenylendiisocyanat als Pfropfgrundlage zu verwenden.

Die o.g. Polyester, Polycarbonate oder Polyurethane können bis zu 500, bevorzugt bis zu 100 Polyalkylenoxideinheiten enthalten, wobei die Polyalkylenoxideinheiten sowohl aus Homopolymeren als auch aus Copolymeren unterschiedlicher Alkylenoxide bestehen können.

Bevorzugt werden wasserlösliche bzw. wasserdispergierbare Pfropfpolymerisate verwendet, die dadurch gekennzeichnet sind, daß sie erhältlich sind durch Polymerisation von
a) mindestens einem Vinylester von aliphatischen C₁-C₂₄-Carbonsäuren, in Gegenwart von
b) Polyethern der allgemeinen Formel I,
in der die Variablen unabhängig voneinander folgende Bedeutung haben:
- R¹: Wasserstoff, C₁-C₂₄-Alkyl, R⁹-C(=O)-, Polyalkoholrest;
- R⁸: Wasserstoff, C₁-C₂₄-Alkyl, R⁹-C(=O)-;
- R² bis R⁴: -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
- R⁹: C₁-C₂₄-Alkyl;
- R¹⁰: Wasserstoff, C₁-C₂₄-Alkyl, R⁹-C(=O)-;
- n: 1 bis 8;
- s: 0;
- u: 1 bis 5000;
- v: 0 bis 5000;
- w: 0 bis 5000.

Besonders bevorzugt werden Pfropfpolymerisate verwendet, die dadurch gekennzeichnet sind, daß sie erhältlich sind durch Polymerisation von
a) mindestens einem Vinylester von aliphatischen C₁-C₁₂-Carbonsäuren, in Gegenwart von
b) Polyethern der allgemeinen Formel I mit einem mittleren Molekulargewicht von 300 bis 100.000 (nach dem Zahlenmittel), in der die Variablen unabhängig voneinander folgende Bedeutung haben:
   - R¹: Wasserstoff, C₁-C₁₂-Alkyl, Polyalkoholrest;
   - R⁸: Wasserstoff, C₁-C₁₂-Alkyl;
   - R² bis R⁴: -(CH₂)₂-, -(CH₂)₃-, -(CH₂)₄-, -CH₂-CH(CH₃)-,
   -CH₂-CH(CH₂-CH₃)-, -CH₂-CHOR¹⁰-CH₂-;
   - R¹⁰: Wasserstoff, C₁-C₁₂-Alkyl;
   - n: 1 bis 5;
   - s: 0;
   - u: 2 bis 2000;
   - v: 0 bis 2000;
   - w: 0 bis 2000.

Ganz besonders bevorzugt werden wasserlösliche bzw. wasserdispergierbare Pfropfpolymerisate verwendet, die dadurch gekennzeichnet sind, daß sie erhältlich sind durch Polymerisation von
a) mindestens einem Vinylester von aliphatischen C₁-C₆-Carbonsäuren, insbesondere Vinylacetat, in Gegenwart von
b) Polyethern der allgemeinen Formel I mit einem mittleren Molekulargewicht von 500 bis 20.000 (nach dem Zahlenmittel), in der die Variablen unabhängig voneinander folgende Bedeutung haben:
   - R¹, R⁸: Wasserstoff, C₁-C₆-Alkyl, insbesondere Wasserstoff;
   - R² bis R⁴: -(CH₂)₂-, -(CH₂)₃-, -CH₂-CH(CH₃)-, -CH₂-CHOR¹⁰-CH₂-, insbesondere -(CH₂)₂-;
   - R¹⁰: Wasserstoff, C₁-C₆-Alkyl;
   - n: 1;
   - s: 0;
   - u: 5 bis 500;
   - v: 0 bis 500, insbesondere 0;
   - w: 0 bis 500, insbesondere 0.

Für die Polymerisation in Gegenwart der Polyether der Formel I seien als Komponente a) folgende copolymerisierbare Monomere genannt:

Vinylester von aliphatischen, gesättigten oder ungesättigten C₁-C₂₄-Carbonsäuren, wie z.B. Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure, Lignocerinsäure, Cerotinsäure sowie Melissensäure.

Bevorzugt werden Vinylester der oben genannten C₁-C₁₂-Carbonsäuren, insbesondere der C₁-C₆-Carbonsäuren verwendet.

Selbstverständlich können auch Mischungen der jeweiligen Monomeren aus der Gruppe a) pfropfpolymerisiert werden.

Die hydrophoben Monomere können daneben auch in Mischung mit einem oder mehreren, ebenfalls hydrophoben Comonomeren, beispielsweise schwer verseifbaren Estern von ungesättigten Carbonsäuren und/oder Alkylethern eingesetzt werden, wobei der Anteil dieser zusätzlichen Monomeren auf maximal 30% beschränkt sein sollte. Bevorzugt sind Anteile von 1 bis 20%.

Als zusätzliche Monomere können für die Polymerisation u.a. mindestens eine weitere Komponente c), ausgewählt aus der Gruppe
c₁) C₁-C₂₄-Alkylester von monoethylenisch ungesättigten C₃-C₈-Carbonsäuren;
c₂) C₁-C₂₄-Hydroxyalkylester von monoethylenisch ungesättigten C₃-C₈-Carbonsäuren;
c₃) C₁-C₂₄-Alkyl-Vinylether
c₄) N-Vinyllactame;
c₅) monoethylenisch ungesättigte C₃-C₈-Carbonsäuren
eingesetzt werden.

Als monoethylenisch ungesättigte C₃-C₈-Carbonsäure kommen Acrylsäure, Methacrylsäure, Maleinsäure, Fumarsäure, Itaconsäure oder Aconitsäure in Frage.

Als Alkylreste seien verzweigte oder unverzweigte C₁-C₂₄-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl sowie deren hydroxylierte Derivate genannt.

Bevorzugt sind verzweigte oder unverzweigte C₁-C₄-Alkylketten, insbesondere Methyl, Ethyl, n-Propyl, 1-Methylethyl, n-Butyl, 1-Methylpropyl-, 2-Methylpropyl, 1,1-Dimethylethyl und deren hydroxylierte Derivate zu nennen.

Besonders bevorzugte Monomere c₁-c₃) sind Methyl(meth)acrylat, Ethyl(meth)acrylat, Hydroxymethyl(meth)acrylat, 2-Hydroxyethyl(meth)acrylat, Methylvinylether und Ethylvinylether.

Die hydrophoben Monomeren können daneben auch in Mischung mit einem oder mehreren hydrophilen Comonomeren eingesetzt werden. Verwendbar sind monoethylenisch ungesättigte C₃-C₈-Carbonsäuren wie Acrylsäure, Methacrylsäure, Maleinsäure, Fumarsäure, Itaconsäure, Aconitsäure, daneben auch N-Vinyllactame wie N-Vinylpyrrolidon, N-Vinylimidazol oder N-Vinylcaprolactam.

Bevorzugte hydrophile Comonomeren sind (Meth)acrylsäure und N-Vinylpyrrolidon.

Die K-Werte der Polymerisate sollen im Bereich von 10 bis 200, bevorzugt 15 bis 150, besonders bevorzugt 15 bis 100, ganz besonders bevorzugt im Bereich von 20 und 80 liegen. Der jeweils gewünschte K-Wert läßt sich in an sich bekannter Weise durch die Zusammensetzung der Einsatzstoffe einstellen. Die K-Werte werden bestimmt nach Fikentscher, Cellulosechemie, Bd. 13, S. 58 bis 64 und 71 bis 74 (1932) in N-Methylpyrrolidon bei 25°C und Polymerkonzentrationen, die je nach K-Wert-Bereich zwischen 0,1 Gew.-% und 5 Gew.-% liegen.

Zur Herstellung der Polymerisate können die Monomeren der Komponente a) in Gegenwart der Polyether sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Die Polymerisation kann beispielsweise eine Lösungspolymerisation, Polymerisation in Substanz, Emulsionspolymerisation, umgekehrte Emulsionspolymerisation, Suspensionspolymerisation, umgekehrte Suspensionspolymerisation oder Fällungspolymerisation sein, ohne daß die verwendbaren Methoden darauf beschränkt sind.

Bei der bevorzugt durchgeführten Polymerisation in Substanz kann man so vorgehen, daß man das Polyalkylenoxid in mindestens einem Monomer der Gruppe a) löst und nach Zugabe eines Polymerisationsinitiators die Mischung auspolymerisiert. Die Pfropfpolymerisation kann auch halbkontinuierlich durchgeführt werden, indem man zunächst einen Teil, z.B. 10 % des zu polymerisierenden Gemisches aus Polyalkylenoxid, mindestens einem Monomeren der Gruppe a) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Pfropfpolymerisate können auch dadurch erhalten werden, daß man die Polyalkylenoxide der Gruppe b) in einem Reaktor vorlegt, auf die Polymerisationstemperatur erwärmt und mindestens ein Monomer der Gruppe a) und Polymerisationsinitiator entweder auf einmal, absatzweise oder vorzugsweise kontinuierlich zufügt und polymerisiert.

Das Mengenverhältnis der als Pfropfgrundlage verwendeten Polyether zu den eingesetzten Vinylestern liegt im Bereich von 1 : 0,5 bis 1 : 50, bevorzugt im Bereich von 1 : 1,5 bis 1 : 35, besonders bevorzugt im Bereich von 1 : 2 bis 1 : 30.

Als Polymerisationsinitiatoren eignen sich vor allem organische Peroxide, wie Diacetylperoxid, Dibenzoylperoxid, Succinylperoxid, Di-tert.-butylperoxid, tert.-Butylperbenzoat, tert.-Butylperpivalat, tert.-Butylpermaleinat, Cumolhydroperoxid, Diisopropylperoxidicarbamat, Bis-(o-toluyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid sowie Mischungen der genannten Initiatoren, Redoxinitiatoren und Azostarter.

Die verwendeten Mengen an Initiator bzw. Initiatorgemischen bezogen auf eingesetztes Monomer liegen zwischen 0,01 und 10 Gew.-%, vorzugsweise zwischen 0,3 und 5 Gew.-%.

Die Pfropfpolymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 5 bar ablaufen.

Falls gewünscht, kann die oben beschriebene Pfropfpolymerisation auch in einem Lösemittel durchgeführt werden. Geeignete Lösemittel sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Die Pfropfpolymerisation kann auch in Wasser als Lösemittel durchgeführt werden. In diesem Fall liegt zunächst eine Lösung vor, die in Abhängigkeit von der Menge der zugegebenen Monomeren der Komponente a) in Wasser mehr oder weniger gut löslich ist. Um wasserunlösliche Produkte, die während der Polymerisation entstehen können in Lösung zu überführen, kann man beispielsweise organische Lösemittel zusetzen, wie einwertige Alkohole mit 1 bis 3 Kohlenstoffatomen, Aceton oder Dimethylformamid. Man kann jedoch auch bei der Pfropfpolymerisation in Wasser so verfahren, daß man die wasserunlöslichen Pfropfpolymerisate durch Zugabe üblicher Emulgatoren oder Schutzkolloide, z.B. Polyvinylalkohol, in eine feinteilige Dispersion überführt.

Als Emulgatoren verwendet man beispielsweise ionische oder nichtionische Tenside, deren HLB-Wert im Bereich von 3 bis 13 liegt. Zur Definition des HLB-Werts wird auf die Veröffentlichung von W.C. Griffin, J. Soc. Cosmetic Chem., Band 5, 249 (1954) hingewiesen.

Die Menge an Tensiden, bezogen auf das Pfropfcopolymerisat, beträgt 0,1 bis 5 Gew.%. Bei Verwendung von Wasser als Lösemittel erhält man Lösungen bzw. Dispersionen der Pfropfpolymerisate. Sofern man Lösungen des Pfropfpolymerisates in einem organischen Lösemittel herstellt bzw. in Mischungen aus einem organischen Lösemittel und Wasser, so verwendet man pro 100 Gew.-Teile des Pfropfpolymerisates 5 bis 200, vorzugsweise 10 bis 100 Gew.-Teile des organischen Lösemittels oder des Lösemittelgemisches.

Zur Erhöhung der Hydrophilie der erfindungsgemäß verwendeten Polymeren können die Estergruppen nach der Polymerisation verseift bzw. teilweise verseift werden. Die Verseifung erfolgt in an sich bekannter Weise durch Zugabe einer Base, bevorzugt durch Zugabe einer methanolischen Natrium- oder Kaliumhydroxidlösung bei Temperaturen im Bereich von 10 bis 50°C, bevorzugt im Bereich von 15 bis 30°C. Der Verseifungsgrad hängt ab von der Menge der eingesetzten Base, von der Verseifungstemperatur und der Verseifungszeit.

Der Verseifungsgrad der Polyvinylestergruppen kann also im Bereich von 0 bis 100 % liegen. Bevorzugt liegt er im Bereich von 20 bis 100 %, besonders bevorzugt im Bereich von 40 bis 100 %, insbesondere von 65 bis 100 % und ganz besonders bevorzugt im Bereich von 80 bis 100 %.

Der Feststoffgehalt der erhaltenen wäßrigen Polymerisat-Dispersionen bzw. Lösungen beträgt in der Regel 10 bis 70 Gew.-%, bevorzugt 15 bis 65 Gew.-%, besonders bevorzugt 20 bis 60 Gew.-%.

Je nach Verseifungsgrad und Konzentration erhält man wäßrige Dispersionen oder Lösungen der erfindungsgemäß verwendeten Polymerisate mit einer Viskosität kleiner 1000 mPas, bevorzugt mit einer viskosität von 5 bis 400 mPas, besonders bevorzugt von 10 bis 250 mPas bei einer Polymerisatkonzentration von 20 Gew.-%.

Die Polymerisat-Dispersionen oder Lösungen können durch verschiedene Trocknungsverfahren wie z.B. Sprühtrocknung, Fluidized Spray Drying, Walzentrocknung oder Gefriertrocknung in Pulverform überführt werden. Durch die vorteilhafte geringe Viskosität der Polymerlösungen bzw. Dispersionen wird als Trocknungsverfahren bevorzugt die Sprühtrocknung eingesetzt. Aus dem so erhaltenen Polymer-Trockenpulver läßt sich durch Redispergieren in Wasser erneut eine wäßrige Dispersion bzw. Lösung herstellen läßt. Die Überführung in Pulverform hat den Vorteil einer besseren Lagerfähigkeit, einer einfacheren Transportmöglichkeit sowie eine geringere Neigung für Keimbefall.

Die erfindungsgemäßen wasserlöslichen oder wasserdispergierbaren Polyalkylenoxid- bzw. Polyglycerin-haltigen Polymerisate eignen sich hervorragend als magensaftlösliche oder als im Magensaft dispergierbare Filmbildner, Bindemittel, Benetzungshilfsstoff und/oder Löslichkeitsverbesserer für pharmazeutische Darreichungsformen.

Aufgrund der enormen Flexibilität und der niedrigen Viskosität sind in der Regel keine zusätzlichen Weichmacher erforderlich.

Gegenstand der Erfindung sind daher auch pharmazeutische Darreichungsformen, enthaltend mindestens ein wasserlösliches oder wasserdispergierbares Pfropfpolymerisat als Überzugsmittel, Bindemittel und/oder filmbildender Hilfsstoff, wobei das Pfropfpolymerisat erhältlich ist durch Polymerisation von
a) mindestens einem Vinylester von aliphatischen C₁-C₂₄-Carbonsäuren, in Gegenwart von
b) Polyethern der allgemeinen Formel I, 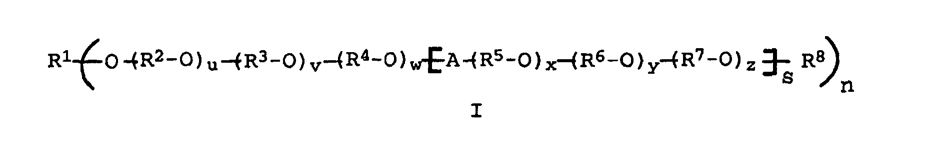
in der die Variablen unabhängig voneinander folgende Bedeutung haben:
- R¹: Wasserstoff, C₁-C₂₄-Alkyl, R⁹-C(=O)-, R⁹-NH-C(=O)-, Polyalkoholrest;
- R⁸: Wasserstoff, C₁-C₂₄-Alkyl, R⁹-C(=O)-, R⁹-NH-C(=O)-;
- R² bis R⁷: -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
- R⁹: C₁-C₂₄-Alkyl;
- R¹⁰: Wasserstoff, C₁-C₂₄-Alkyl, R⁹-C(=O)-;
- A: -C(=O)-O-, -C(=O)-B-C(=O)-O-,
-C(=O)-NH-B-NH-C(=O)-O-;
- B: -(CH₂)ₜ-, Arylen, gegebenenfalls substituiert;
- n: 1 bis 8;
- s: 0 bis 500;
- t: 1 bis 12;
- u: 1 bis 5000;
- v: 0 bis 5000;
- w: 0 bis 5000;
- x: 1 bis 5000;
- y: 0 bis 5000;
- z: 0 bis 5000, dadurch gekennzeichnet, dass das Molekulargewicht der Polyether im Bereich von 300 bis 100 000 liegt.

Bezüglich einer näheren Erläuterung der Pfropfpolymerisate, einschließlich der variablen und deren bevorzugte Ausführungsformen, sei auf die bereits eingangs erfolgte Beschreibung hingewiesen

Bei den überzogenen Darreichungsformen handelt es sich bevorzugt u.a. um Filmtabletten, Filmmikrotabletten, Dragees, überzogene Pastillen, Kapseln, Kristalle, Granulate oder Pellets.

Bei den bindemittelhaltigen Darreichungsformen handelt es sich bevorzugt u.a. um Tabletten, Mikrotabletten, Kerne, Granulate oder Pellets.

Weiterhin können die erfindungsgemäßen Polymere zur Herstellung von Lösungen und Sprays verwendet werden, die, auf Haut oder Schleimhaut aufgebracht, einen Film ausbilden. Bedingt durch die enorme Dehnbarkeit und Adhäsivität haften die Filme lange auf der Haut oder Schleimhaut. Die Applikationsfrequenz kann so reduziert werden und der Tragekomfort ist erhöht. Beispiele hierfür sind Sprühverbände für Wunden, Desinfektionssprays, Lösungen mit Mycostatica, Mundsprays oder -lösungen mit Antibiotika etc.. Aufgrund der Flexibilität ist auch der Einsatz bei transdermalen therapeutischen Systemen vorteilhaft.

Die erfindungsgemäß verwendeten Pfropfpolymerisate benetzen leicht lipophile Oberflächen und besitzen hervorragende Schutzkolloideigenschaften. Eingearbeitet in Suspensionen und Emulsionen lagern sie sich an die Teilchen der dispersen Phase und stabilisieren diese. Sie können daher als Benetzungshilfsmittel und Stabilisatoren in dispersen Systemen verwendet werden.

Durch Wechselwirkung mit schwer wasserlöslichen Arzneistoffen verbessern sie deren Löslichkeit und Lösungsgeschwindigkeit, wodurch Resorbierbarkeit und Bioverfügbarkeit der Arzneistoffe verbessert werden. Diese vorteilhafte Wirkung zeigt sich beispielsweise bei den Darreichungsformen, bei denen der Wirkstoff nicht gelöst vorliegt, wie z.B. Tabletten, Granulate, Suspensionen etc..

Die erfindungsgemäß verwendeten Polymere können gegebenenfalls auch in Kombination mit anderen Hilfsstoffen zusammen mit Wirkstoffen zu Polymer-Wirkstoffschmelzen verarbeitet werden, die entweder zu Arzneistoffen extrudiert und kalandriert werden oder nach der Extrusion zu Granulaten oder Pulvern zerteilt werden und erst anschließend in Arzneiformen verarbeitet werden, beispielsweise zu Tabletten verpreßt werden. Dabei bringen die Pfropfpolymerisate die bereits oben aufgeführten Eigenschaften in die Darreichungsform ein.

In verschiedenen pharmazeutischen Darreichungsformen können die erfindungsgemäßen Polymere folgende Funktionen hervorragend erfüllen:

Dispergierhilfsstoff, Suspendierhilfsstoff, Benetzungsmittel, Solubilisator für schwerlösliche Arzneistoffe, Emulgator, Kristallisationsinhibitor, Anticakinghilfsstoff,Schutzkolloid, Bioadhäsivum zur Verlängerung und Intensivierung des Kontaktes mit der Schleimhaut, Spreithilfsmittel, Viskositätsregulator, Hilfsstoff zur Herstellung von festen Lösungen mit Arzneistoffen, Hilfsstoff zur Einstellung der Wirkstofffreisetzung in Retardformulierungen.

Die in Wasser nicht oder nur gering löslichen aber dispergierbaren erfindungsgemäßen Polymere können auch als Retardierungspolymere sowie als Klebstoffe für Wirkstoffpflaster verwendet werden.

Bei der Verwendung zur Herstellung von Suppositorien und Vaginalglobuli gewährleisten die Polymere einerseits die Flexibilität der Darreichungsform und fördern andererseits den Zerfall und die Wirkstoffauflösung und sie kleiden die Schleimhaut mit einem wirkstoffhaltigen Film aus, der die Resorption verstärkt.

Wie Tabelle 1 zeigt, weisen die wäßrigen Lösungen der erfindungsgemäßen verseiften bzw. teilverseiften Polymerisate eine deutlich niedrigere Viskosität auf als entsprechende Lösungen von Hydroxypropylmethylcellulose.

**Tabelle 1:**

| Polymerisate | Viskosität (20 gew.-%ige wäßr. Lösung) [mPas] | Flexibilität Reißdehnung [%] |
|---|---|---|
| | | |
| PEG 6000/VAc (Beispiel 1) | 124 | 74 |
| PEG 6000/VAc (Beispiel 2) | 181 | 172 |
| PEG 9000/VAc (Beispiel 3) | 199 | 225 |
| Polyglycerin 2200/VAc (Beispiel 4) | 199 | 313 |
| Lutrol® F 68/VAc (Beispiel 5) | 145 | 110 |
| PEG 6000/VAc/MMA (Beispiel 6) | 144 | 122 |
| Pharmacoat® 606 (Vergleich) | 5168 | 15 |

Somit können beim Coaten von Tabletten mit den Polymerisatdispersionen ebenso wie bei Bindemittelanwendungen konzentriertere Polymerzubereitungen eingesetzt werden, wodurch sich die Verfahren wesentlich kostengünstiger und zeitsparender gestalten lassen.

Die Auflösung bzw. Redispergierung der pulver- oder granulatförmigen Polymerisate zu wäßrigen Dispersionen bzw. Lösungen erfolgt wesentlich schneller als bei anderen Filmbildnern oder Bindemitteln, da die erfindungsgemäßen Polymerisate gut von Wasser benetzt werden, wenig klumpen und eine sehr hohe Auflösungsgeschwindigkeit aufweisen.

Magensaftlösliche Tabletten, die mit den erfindungsgemäßen verseiften bzw. teilverseiften Polymerisaten gecoatet wurden, zeigen eine gegenüber dem Kern nur geringfügig verlängerte Zerfallszeit, d.h. der Filmüberzug löst sich sehr schnell in künstlichem Magensaft auf.

Im Fall von Hydroxypropylmethylcellulose, Typ Pharmacoat 606 als Coatingmaterial ist der Zerfall deutlich verlängert (Siehe Beispiele 7 und 8 mit den jeweiligen Vergleichsbeispielen). Ferner wird durch die erfindungsgemäße Verwendung der Polymerisate die mechanische Festigkeit der Tabletten im Vergleich zu Hydroxypropylmethylcellulose sehr viel stärker erhöht.

Tabletten quellen in Abhängigkeit von den verwendeten Hilfsund Wirkstoffen, der Lagerzeit und den Lagerbedingungen, wie Temperatur und Feuchtigkeit, unterschiedlich stark. Ein starrer Filmüberzug erleidet bei Quellung des Kernes Risse. Deshalb ist die Elastizität von Filmbildnern eine wichtige Größe. Pfropfpolymerisate besitzen eine ausgesprochen hohe Flexibilität und Elastizität. So kann die Reißdehnung bis zu 300 % betragen. Eine Rißbildung ist daher auch bei starker Kernquellung nicht zu erwarten.

Die Pfropfpolymere können in reiner Form oder aber zusammen mit den üblichen Hilfsstoffen auf den wirkstoffhaltigen Kern appliziert werden. Übliche Hilfsstoffe sind z.B. Farbpigmente zur Einfärbung, Weißpigmente, wie Titandioxid, zur Erhöhung der Deckkraft, Talkum und Siliciumdioxid als Antiklebemittel, Polyethylenglykole, Glycerin, Propylenglykol, Triacetin, Triethylcitrat als Weichmacher und verschiedene oberflächenaktive Stoffe, wie Natriumlaurylsulfat, Polysorbat 80, Pluronics und Cremophore, zur Verbesserung des Benetzungsverhaltens. Die exemplarisch genannten Stoffe stellen keine Begrenzung dar. Es können alle bekanntermaßen für magensaftlösliche Filmüberzüge geeigneten Zusatzstoffe verwendet werden.

Es ist ferner möglich, die erfindungsgemäß verwendeten Polymerisate mit anderen Filmbildnern bzw. Polymeren im Verhältnis 1:9 bis 9:1 zu kombinieren.

Hierzu können beispielsweise folgende Polymere eingesetzt werden:

Polyvinylpyrrolidon, Polyvinylpyrrolidon-Copolymere, wasserlösliche Cellulosederivate, wie Hydroxypropylcellulose, Hydroxypropylmethylcellulose, Methylcellulose, Hydroxyethylcellulose, Acrylat- und Methacrylat-Copolymere, Polyvinylalkohole, Polyethylenglykole, Polyethylenoxid-Polypropylenoxid-Blockpolymere.

Als Überzugsverfahren lassen sich die gebräuchlichen Verfahren, wie das Coaten in der Wirbelschicht oder im Horizontaltrommelcoater, das Tauchschwertverfahren und das Kesselcoatingverfahren, anwenden. Neben der Anwendung auf Tabletten können die erfindungsgemäßen Polymere auch für das Coating von anderen pharmazeutischen Zubereitungen, wie Granulaten, Pellets, Kristallen oder Kapseln, eingesetzt werden. Die neuen Überzugsmittel werden wie üblich in einer Stärke von 5 bis 200 µm, vorzugsweise 10 bis 100 µm, aufgetragen.

Bei der Verwendung als Bindemittel unterscheidet man je nach Verarbeitungsverfahren zwischen Feucht- und Trockenbindemittel. Letztere werden u.a. bei der Direkttablettierung und bei der Trockengranulation bzw. Kompaktierung verwendet. Hierbei wird das Bindemittel mit dem Wirkstoff und gegebenenfalls weiteren Hilfsstoffen vermischt und anschließend direkttablettiert oder granuliert bzw. kompaktiert.

Im Gegensatz dazu wird bei der Feuchtgranulation die Wirkstoff-Hilfsstoffmischung mit einer Lösung des Bindemittels in Wasser oder einem organischen Lösungsmittel befeuchtet, die feuchte Masse durch ein Sieb gegeben und anschließend getrocknet. Befeuchtung und Trocknung können dabei auch parallel ablaufen, wie z.B. in der Wirbelschichtgranulation.

Für eine optimale Verarbeitung soll das Bindemittel in Lösung niedrigviskos sein, da viskose Lösungen zu inhomogenen Granulaten führen.

Ein Bindemittel soll zu gleichmäßigen, harten, abriebsstabilen Granulaten bzw. Tabletten führen. Insbesondere bei Tabletten kommt der Bruchfestigkeit besondere Bedeutung zu, da sich viele Wirkstoffe schlecht verpressen lassen und somit Tabletten mit unzureichender mechanischer Stabilität ergeben.

Weiterhin soll der Zerfall der Arzneiformen sowie die Freisetzungsgeschwindigkeit der Wirkstoffe durch das Bindemittel nicht nennenswert nachteilig beeinflußt werden.

Die gängigsten Bindemittel sind beispielsweise Polyvinylpyrrolidon, Vinylacetat-Vinylpyrrolidon Copolymere, Gelatine, Stärkekleister, Maltodextrine, hydroxyalkylierte bzw. carboxyalkylierte Cellulosederivate, wie Hydroxypropylmethylcellulose, Methylcellulose, Natriumcarboxymethylcellulose sowie natürliche Gummisorten, wie beispielsweise Gummi Arabicum, Pektin oder Alginat.

Viele dieser Bindemittel weisen in Lösung eine hohe Viskosität auf und sind schlecht verarbeitbar. Durch die hohe Viskosität werden die zu granulierenden Pulverteilchen schlecht und ungleichmäßig benetzt, so daß daraus eine zu geringe Granulatfestigkeit und eine ungünstige Korngrößenverteilung resultieren.

Viele Bindemittel sind zudem hygroskopisch und quellen bei Wasseraufnahme. Dadurch können sich die Granulat- und Tabletteneigenschaften dramatisch verändern.

Überraschenderweise wurde nun gefunden, daß die erfindungsgemäßen Polymerisate über ausgezeichnete Bindemittelwirkungen verfügen und zudem in Konzentrationsbereichen von 0,5 bis 20 Gew.-%, bevorzugt 1 bis 10 Gew.-% der Gesamtmenge der Formulierung den Zerfall nicht nennenswert beeinflussen. Aufgrund des guten Benetzungsverhaltens der Pfropfpolymerisate kann sich zudem die Freisetzung von schlecht löslichen Wirkstoffen verbessern.

Mit den Pfropfpolymeren als Bindemittel ergeben sich mechanisch außerordentlich stabile und auch über lange Lagerzeiten stabile Granulate bzw. Tabletten.

Gegenstand der Erfindung sind auch wasserlösliche bzw. wasserdispergierbare Pfropfpolymerisate, die erhältlich sind durch Polymerisation von
a) mindestens einem Vinylester von aliphatischen C₁-C₂₄-Carbonsäuren, in Gegenwart von
b) Polyethern der allgemeinen Formel I, 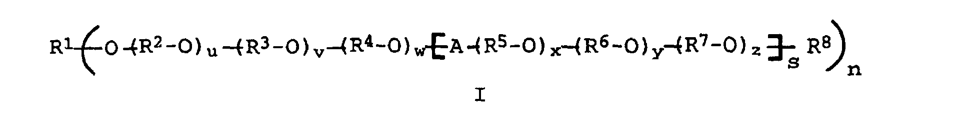
in der die Variablen unabhängig voneinander folgende Bedeutung haben:
- R¹: Wasserstoff, C₁-C₂₄-Alkyl, R⁹-C(=O)-, Polyalkoholrest;
- R⁸: Wasserstoff, C₁-C₂₄-Alkyl, R⁹-C(=O)-;
- R² bis R⁷: -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
- R⁹: C₁-C₂₄-Alkyl;
- R¹⁰: Wasserstoff, C₁-C₂₄-Alkyl, R⁹-C(=O)-;
- A: -C(=O)-O-, -C(=O)-B-C(=O)-O-;
- B: -(CH₂)ₜ-, Arylen, gegebenenfalls substituiert;
- n: 1 bis 8;
- s: 1 bis 500;
- t: 1 bis 12;
- u: 1 bis 5000;
- v: 0 bis 5000;
- w: 0 bis 5000;
- x: 1 bis 5000;
- y: 0 bis 5000;
- z: 0 bis 5000. dadurch gekennzeichnet, dass das Molekulargewicht der Polyether im Bereich von 300 bis 100 000 liegt.

Gegenstand der Erfindung sind weiterhin wasserlösliche bzw. wasserdispergierbare Pfropfpolymerisate, die erhältlich sind durch Polymerisation von
a) mindestens einem Vinylester von aliphatischen C₁-C₂₄-Carbonsäuren, in Gegenwart von
b) Polyethern der allgemeinen Formel Ia, 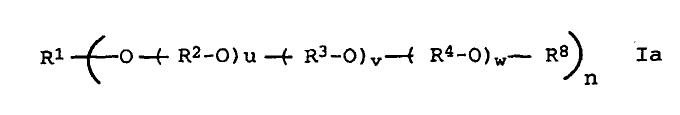 in der die Variablen unabhängig voneinander folgende Bedeutung haben:
   - R¹: Wasserstoff, C₁-C₂₄-Alkyl, R⁹-C(=O)-, Polyalkoholrest;
   - R⁸: Wasserstoff, C₁-C₂₄-Alkyl, R⁹-C(=O)-;
   - R² bis R⁴: -(CH₂)₂-, -(CH₂)₃-, -(CH₂)₄-, -CH₂-CH(CH₃)-,
   -CH₂-CH(CH₂-CH₃)-;
   - R⁹: C₁-C₂₄-Alkyl;
   - n: 1 bis 8;
   - u: 1 bis 5000;
   - v: 0 bis 5000;
   - w: 0 bis 5000
   zusammen mit
c) mindesten einem Monomeren, ausgewählt aus der Gruppe
   c₁) C₁-C₂₄-Alkylester von monoethylenisch ungesättigten C₃-C₈-Carbonsäuren;
   c₂) C₁-C₂₄-Hydroxyalkylester von monoethylenisch ungesättigten C₃-C₈-Carbonsäuren;
   c₃) C₁-C₂₄-Alkyl-Vinylether;
   c₄) N-Vinyllactame;
   c₅) monoethylenisch ungesättigte C₃-C₈-Carbonsäuren.

Bevorzugt sind Pfropfpolymerisate, die erhältlich sind durch Polymerisation von
a) mindestens einem Vinylester von aliphatischen C₁-C₁₂-Carbonsäuren, in Gegenwart von
b) Polyethern der allgemeinen Formel Ia, in der die Variablen unabhängig voneinander folgende Bedeutung haben:
   - R¹: Wasserstoff, C₁-C₁₂-Alkyl, Polyalkoholrest;
   - R⁸: Wasserstoff, C₁-C₁₂-Alkyl;
   - R² bis R⁴: -(CH₂)₂-, -(CH₂)₃-, -(CH₂)₄-, -CH₂-CH(CH₃)-,
   -CH₂-CH(CH₂-CH₃)-;
   - n: 1 bis 5;
   - u: 2 bis 2000;
   - v: 0 bis 2000;
   - w: 0 bis 2000
   zusammen mit
c) mindestens einem Monomeren, ausgewählt aus der Gruppe
   c₁) C₁-C₁₂-Alkylester von monoethylenisch ungesättigten C₃-C₈-Carbonsäuren;
   c₂) C₁-C₁₂-Hydroxyalkylester von monoethylenisch ungesättigten C₃-C₈-Carbonsäuren;
   c₃) C₁-C₁₂-Alkyl-Vinylether;
   c₄) N-Vinyllactame;
   c₅) monoethylenisch ungesättigte C₃-C₈-Carbonsäuren.

Ganz besonders bevorzugt sind Pfropfpolymerisate, die erhältlich sind durch Pfropfen von
a) mindestens einem Vinylester von aliphatischen C₁-C₆-Carbonsäuren, in Gegenwart von
b) Polyethern der allgemeinen Formel Ia, in der die Variablen unabhängig voneinander folgende Bedeutung haben:
   - R¹ und R⁸: Wasserstoff, C₁-C₆-Alkyl;
   - R² bis R⁴: -(CH₂)₂-, -(CH₂)₃-, -(CH₂)₄-, -CH₂-CH(CH₃)-,
   -CH₂-CH(CH₂-CH₃)-;
   - n: 1;
   - u: 2 bis 500;
   - v: 0 bis 500;
   - w: 0 bis 500
   zusammen mit
c) mindestens einem Monomeren, ausgewählt aus der Gruppe
   c₁) C₁-C₆-Alkylester von monoethylenisch ungesättigten C₃-C₈-Carbonsäuren;
   c₄) N-Vinylpyrrolidon, N-Vinylimidazol, N-Vinylcaprolactam;
   c₅) (Meth)acrylsäure.

Gegenstand der Erfindung sind ebenfalls wasserlösliche bzw. wasserdispergierbare Pfropfpolymerisate, die erhältlich sind durch Polymerisation von
a) mindestens einem Vinylester von aliphatischen C₁-C₂₄-Carbonsäuren in Gegenwart von
b) Polyglycerin der allgemeinen Formel Ib, 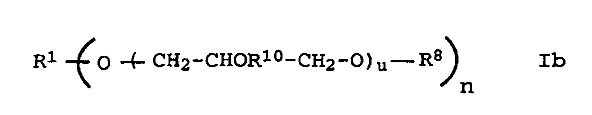
in der die Variablen unabhängig voneinander folgende Bedeutung haben:
- R¹: Wasserstoff, C₁-C₂₄-Alkyl, R⁹-C(=O)-, Polyalkoholrest;
- R⁸ und R¹⁰: Wasserstoff, C₁-C₂₄-Alkyl, R⁹-C(=O)-;
- R⁹: C₁-C₂₄-Alkyl;
- n: 1 bis 8;
- u: 1 bis 2000.

Bevorzugt sind Pfropfpolymerisate, die erhältlich sind durch Polymerisation von
a) mindestens einem Vinylester von aliphatischen C₁-C₁₂-Carbonsäuren in Gegenwart von
b) Polyglycerin der allgemeinen Formel Ib, in der die Variablen unabhängig voneinander folgende Bedeutung haben:
   - R¹: Wasserstoff, C₁-C₁₂-Alkyl, Polyalkoholrest;
   - R⁸ und R¹⁰: Wasserstoff, C₁-C₁₂-Alkyl;
   - n: 1 bis 5;
   - u: 1 bis 500.

Besonders bevorzugt sind Pfropfpolymerisate, die erhältlich sind durch Polymerisation von
a) mindestens einem Vinylester von aliphatischen C₁-C₆-Carbonsäuren in Gegenwart von
b) Polyglycerin der allgemeinen Formel Ib, in der die Variablen unabhängig voneinander folgende Bedeutung haben:
   - R¹, R⁸ und R¹⁰: Wasserstoff, C₁-C₆-Alkyl;
   - n: 1;
   - u: 1 bis 100.

Neben den linearen Polyglycerinen der allgemeinen Formel II können auch verzweigte und/oder cyclische Polyglycerine als Pfropfgrundlage verwendet werden.

Die Pfropfpolymerisate auf Basis von Polyglycerin sind dadurch gekennzeichnet, daß für die Pfropfung zusätzlich zu den Vinylestern mindestens ein weiteres Monomer c), ausgewählt aus der Gruppe
c₁) C₁-C₂₄-Alkylester von monoethylenisch ungesättigten C₃-C₈-Carbonsäuren;
c₂) C₁-C₂₄-Hydroxyalkylester von monoethylenisch ungesättigten C₃-C₈-Carbonsäuren;
c₃) C₁-C₂₄-Alkyl-Vinylether;
c₄) N-Vinyllactame;
c₅) monoethylenisch ungesättigte C₃-C₈-Carbonsäuren
verwendet werden kann.

Bevorzugte Monomere c) sind C₁-C₆-Alkylester von monoalkylenisch ungesättigten C₃-C₈-Carbonsäuren, N-Vinylpyrrolidon, N-Vinylimidazol, N-Vinylcaprolactam und (Meth)acrylsäure.

Bezüglich einer näheren Erläuterung der Pfropfpolymerisate auf Basis der Polyether der Formel Ia sowie auf Basis von Polyglycerin der Formel Ib, einschließlich der bevorzugten Ausführungsformen, sei auf die bereits eingangs erfolgte Beschreibung hingewiesen

In den nachfolgenden Beispielen wird die Herstellung und Verwendung der erfindungsgemäßen Pfropfpolymerisate näher erläutert, ohne die Erfindung jedoch auf die Ausführungsbeispiele einzuschränken.

### Herstellung der Pfropfpolymerisate

### Beispiel 1

In einem Polymerisationsgefäß wurden 72 g Polyethylenglykol (mittleres Molekulargewicht 6000, Pluriol® E 6000) vorgelegt und unter Rühren und leichtem Stickstoffstrom auf 80°C erhitzt. Unter Rühren wurde unter Aufrechterhaltung der 80°C ein Zulauf von 410 g Vinylacetat in 3 h und gleichzeitig ein weiterer Zulauf von einer Lösung vom 1,4 g tert.-Butylperpivalat in 30 g Methanol ebenfalls in 3 h zugetropft. Nach vollständiger Zugabe wurde noch 2 h bei 80°C gerührt. Nach dem Abkühlen wurde das Polymerisat in 450 ml Methanol gelöst. Zur Verseifung wurden bei 30°C 50 ml einer 10%igen methanolischen Natriumhydroxidlösung zugegeben. Nach ca. 40 min. wurde die Reaktion durch Zugabe von 750 ml 1%iger Essigsäure abgebrochen. Zur Entfernung des Methanols wurde die Lösung wasserdampfdestilliert. Nach anschließender Gefriertrocknung der klaren Lösung erhielt man ein weißes Pulver. Eine 20% wäßrige Lösung des erhaltenen Polymeren wies eine Viskosität von 124 mPas auf. Der K-Wert lag bei 54, die Reißdehnung betrug 74 %.

### Beispiel 2

In einem Polymerisationsgefäß wurden 30 g Polyethylenglykol (mittleres Molekulargewicht 6000, Pluriol E 6000) vorgelegt und unter Rühren und leichtem Stickstoffstrom auf 80°C erhitzt. Unter Rühren wurde unter Aufrechterhaltung der 80°C ein simultaner Zulauf von 570 g Vinylacetat und einer Lösung vom 2 g tert.-Butylperpivalat in 45 g Methanol ebenfalls in 3 h zugetropft. Nach vollständiger Zugabe wurde noch 2 h bei 80°C gerührt. Nach dem Abkühlen wurde das Polymerisat in 550 ml Methanol gelöst.
Zur Verseifung wurden bei 30°C 65 ml einer 10 Gew.-%igen methanolischen Natriumhydroxidlösung zugegeben. Nach ca. 40 min. wurde die Reaktion durch Zugabe von 500 ml 1%iger Essigsäure abgebrochen. Zur Entfernung des Methanols wurde die Lösung wasserdampfdestilliert. Nach anschließender Gefriertrocknung der klaren Lösung erhielt man ein weißes Pulver. Eine 20 Gew.-%ige wäßrige Lösung des erhaltenen Polymeren wies eine Viskosität von 46 mPas auf. Der K-Wert betrug 73, die Reißdehnung lag bei 171 %.

### Beispiel 3

In einem Polymerisationsgefäß wurden 100 g Polyethylenglykol (mittleres Molekulargewicht 9000) vorgelegt und unter Rühren und leichtem Stickstoffstrom auf 80°C erhitzt. Unter Rühren wurde unter Aufrechterhaltung der 80°C ein simultaner Zulauf von 300 g vinylacetat und einer Lösung vom 1,5 g tert.-Butylperpivalat in 30 ml Methanol in 3 h zugetropft. Nach vollständiger Zugabe wurde noch 2 h bei 80°C nachpolymerisiert. Nach dem Abkühlen wurde das Polymerisat in 400 ml Methanol gelöst. Zur Verseifung wurden bei 30°C 40 ml einer 10 Gew.-%igen methanolischen Natriumhydroxidlösung zugegeben. Nach ca. 40 min. wurde die Reaktion durch Zugabe von 550 ml 1%iger Essigsäure abgebrochen. Zur Entfernung des Methanols wurde die Lösung wasserdampfdestilliert. Nach anschließender Gefriertrocknung der klaren Lösung erhielt man ein weißes Pulver. Eine 20 Gew.-%ige wäßrige Lösung des erhaltenen Polymeren wies eine Viskosität von 199 mPas auf. Der K-Wert betrug 58, die Reißdehnung lag bei 225 %.

### Beispiel 4

In einem Polymerisationsgefäß wurden 60 g Polyglycerin (mittleres Molekulargewicht 2200) vorgelegt und unter Rühren und leichtem Stickstoffstrom auf 80°C erhitzt. Unter Rühren wurde unter Aufrechterhaltung der 80°C ein simultaner Zulauf von 340 g Vinylacetat und einer Lösung vom 1,2 g tert.-Butylperpivalat in 30 ml Methanol in 3 h zugetropft. Nach vollständiger Zugabe wurde noch 2 h bei 80°C nachpolymerisiert. Nach dem Abkühlen wurde das Polymerisat in 400 ml Methanol gelöst. Zur Verseifung wurden bei 30°C 40 ml einer 10 Gew.-%igen methanolischen Natriumhydroxidlösung zugegeben. Nach ca. 40 min. wurde die Reaktion durch Zugabe von 640 ml 1%iger Essigsäure abgebrochen. Zur Entfernung des Methanols wurde die Lösung wasserdampfdestilliert. Nach anschließender Gefriertrocknung der klaren Lösung erhielt man ein weißes Pulver. Eine 20 Gew.-%ige wäßrige Lösung des erhaltenen Polymeren wies eine Viskosität von 199 mPas auf. Der K-Wert betrug 66, die Reißdehnung betrug 313 %.

### Beispiel 5

In einem Polymerisationsgefäß wurden 60 g Polyethylenglykol-Polypropylenglykol-Blockcopolymer (mittleres Molekulargewicht ca. 8000) vorgelegt und unter Rühren und leichtem Stickstoffstrom auf 80°C erhitzt. Unter Rühren wurde unter Aufrechterhaltung der 80°C ein simultaner Zulauf von 340 g Vinylacetat und einer Lösung vom 1,2 g tert.-Butylperpivalat in 30 ml Methanol in 3 h zugetropft. Nach vollständiger Zugabe wurde noch 3 h bei 80°C nachpolymerisiert. Nach dem Abkühlen wurde das Polymerisat in 400 ml Methanol gelöst. Zur Verseifung wurden bei 30°C 40 ml einer 10 Gew.-%igen methanolischen Natriumhydroxidlösung zugegeben. Nach ca. 40 min. wurde die Reaktion durch Zugabe von 650 ml 1%iger Essigsäure abgebrochen. Zur Entfernung des Methanols wurde die Lösung wasserdampfdestilliert. Nach anschließender Gefriertrocknung der klaren Lösung erhielt man ein weißes Pulver. Eine 20 Gew.-%ige wäßrige Lösung des erhaltenen Polymeren wies eine viskosität von 145 mPas auf. Der K-Wert betrug 45, die Reißdehnung lag bei 110 %.

### Beispiel 6

In einem Polymerisationsgefäß wurden 60 g Polyethylenglykol (mittleres Molekulargewicht ca. 6000) vorgelegt und unter Rühren und leichten Stickstoffstrom auf 80°C erhitzt. Unter Rühren wurde unter Aufrechterhaltung der 80°C ein simultaner Zulauf von 332 g Vinylacetat und 8 g Methylmethacrylat in 3 h und gleichzeitig ein weiterer Zulauf von einer Lösung vom 1,2 g tert.-Butylperpivalat in 30 ml Methanol ebenfalls in 3 h zugetropft. Nach vollständiger Zugabe wurde noch 2 h bei 80°C nachpolymerisiert. Nach dem Abkühlen wurde das Polymerisat in 400 ml Methanol gelöst. Zur Verseifung wurden bei 30°C 40 ml einer 10 Gew.-%igen methanolischen Natriumhydroxidlösung zugegeben. Nach ca. 40 min. wurde die Reaktion durch Zugabe von 600 ml 1%iger Essigsäure abgebrochen. Zur Entfernung des Methanols wurde die Lösung wasserdampfdestilliert. Nach anschließender Gefriertrocknung der klaren Lösung erhielt man ein weißes Pulver. Eine 20%ige wäßrige Lösung des erhaltenen Polymeren wies eine Viskosität von 144 mPas auf. Der K-Wert betrug 56, die Reißdehnung 122%.

### Beispiel 7

### Herstellung von Propranolol-HCl Filmtabletten (magensaftlöslicher Überzug)

Auf 9 mm gewölbte Tablettenkerne mit 40 mg Propranolol-HCl (Fa. Knoll AG) , 195,0 mg Ludipress® (Fa. BASF AG), 12, 50 mg Kollidon® VA 64 (Fa. BASF AG) und 2,50 mg Magnesiumstearat wurde in einem Horizontaltrommelcoater (Accela-Cota 24", Fa. Manesty) ein Filmüberzug gemäß folgender Zusammensetzung aufgesprüht:

| | |
|---|---|
| Pfropfpolymer PEG 6000/VAc aus Beispiel 1 | 10,0 Gew.-% |
| Sicovit® rot ( Fa . BASF AG) | 1,5 Gew.-% |
| Titandioxid BN 56 (Fa. Kronos) | 3,0 Gew.-% |
| Talkum Pulver (Fa. Riedel de Haen) | 4,5 Gew.-% |
| Wasser | 81,0 Gew.-% |

Zur Herstellung der Sprühdispersion wurde das Pf ropfpolymerisat in Wasser gelöst, mit Sicovit® rot, Titandioxid und Talkum versetzt und anschließend in einer Korundscheibenmühle homogenisiert. 1260 g (incl. eines Zuschlages von 10 % für Sprühverluste) wurden bei einer Zulufttemperatur von 60°C und einer Sprührate von 30 g/min mit einer 1,0 mm weiten Sprühdüse und einem Sprühdruck von 2,0 bar auf 5 000 g Kerne appliziert. Nach der Sprühung wurde noch 5 min. bei 60°C nachgetrocknet.

Man erhielt sehr glatte, glänzende, rote Filmtabletten mit folgenden Eigenschaften:

| | |
|---|---|
| Aussehen | sehr glatte Oberfläche, schön ausgebildete Gravur |
| Zerfall (künstl. Magensaft) | 5 min. 26 s. |
| Zerfallszeitdifferenz (Filmtablette-Kern) | 57 s. |
| Bruchfestigkeit | 92 N |
| Bruchfestigkeitsdifferenz (Filmtablette-Kern) | 23 N |

### Vergleichsbeispiel

Analog Beispiel 7 wurde anstelle des Pfropfpolymerisats Pharmacoat® 606 (Hydroxypropylmethylcellulose, Fa. Shin-etsu) eingesetzt und wie laut Herstellerangaben empfohlen, 1,0 Gew.-% Polyethylenglykol 6000 (Lutrol® 6000, BASF AG) zugesetzt.

Folgende Tabletteneigenschaften wurden erzielt:

| | |
|---|---|
| Aussehen | leicht rauhe Oberfläche, zugeschmierte Gravur |
| Zerfall (künstl. Magensaft) | 11 min. 12 s. |
| Zerfallszeitdifferenz (Filmtablette-Kern) | 6 min. 43 s. |
| Bruchfestigkeit | 87 N |
| Bruchfestigkeitsdifferenz (Filmtablette-Kern) | 18 N |

### Beispiel 8

Gemäß Beispiel 7 wurde ein Pfropfpolymerisat aus Methyl-PEG 1500/VAc (hergestellt analog Beispiel 1) verarbeitet. Die verwendete Sprühlösung war wie folgt zusammengesetzt:

| | |
|---|---|
| Methyl-PEG 1500/VAc | 20,0 Gew.-% |
| Sicovit® rot (Fa. BASF AG) | 1, 5 Gew.-% |
| Titandioxid BN 56 (Fa. Kronos) | 3,0 Gew.-% |
| Talkum Pulver (Fa. Riedel de Haen) | 4,5 Gew.-% |
| Wasser | 71,0 Gew.-% |

Es wurden wiederum glatte, leicht glänzende, rote Filmtabletten erhalten.

| | |
|---|---|
| Aussehen | glatte Oberfläche, schön ausgebildete Gravur |
| Zerfall (künstl. Magensaft) | 5 min. 35 s. |
| Zerfallszeitdifferenz (Filmtablette-Kern) | 1 min. 06 s. |
| Bruchfestigkeit | 94 N |
| Bruchfestigkeitsdifferenz (Filmtablette-Kern) | 25 N |

### Vergleichsbeispiel

Analog Beispiel 8 wurde anstelle von Methyl-PEG 1500/VAc Pharmacoat® 606 eingesetzt. Aufgrund der enorm hohen Viskosität von Pharmacoat® 606 konnte die Lösung nicht aufgesprüht werden.

### Beispiel 9

### Verwendung als Bindemittel in Glibenclamid-Tabletten

890 g Calciumhydrogenphosphat (Fa. Rhone Poulenc) und 30 g Glibenclamid (Fa. Arzneimittelwerk Dresden) wurden über ein 0,8 mm-Sieb gesiebt und in einem Turbulamischer (Fa. Bachofen) 5 min. gemischt. Diese Pulvermischung wurde in einem Stephanmischer (Fa. Stephan) mit 119 g einer 25 Gew.-%igen wäßrigen Zubereitung eines Pfropfpolymers aus PEG 1500/VAc (hergestellt analog Beispiel 2) unter Rühren langsam befeuchtet. Zur vollständigen Durchfeuchtung wurde nach der Zugabe der Bindemittelzubereitung noch 2 min. bei 800 U/min weitergerührt. Die feuchte Masse wurde anschließend durch ein 0,8 mm-Sieb gegeben und auf einer Horde 20 Std. bei 25°C abgetrocknet. Nach der Zugabe von 45 g Kollidon® CL (Fa. BASF) und 5 g Magnesiumstearat (Fa. Bärlocher) erfolgte die Endmischung wiederum im Turbulamischer über 5 min. Diese Tablettiermischung wurde anschließend auf einer Korsch PH 106 Rundläuferpresse (Fa. Korsch) bei 10 kN und 18 kN Preßkraft zu biplanaren, facettierten Tabletten mit 12 mm Durchmesser und 500 mg Gesamtgewicht verpreßt.

| | | |
|---|---|---|
| Eigenschaften | 10 KN Preßkraft | 18 KN Preßkraft |
| Bruchfestigkeit | 28 N | 53 N |
| Friabilität | 0,7 % | 0 % |
| Zerfall | 29 s. | 37 s. |

### Vergleichsbeispiel

Die Herstellung erfolgte analog Beispiel 9, jedoch mit Hydroxypropylmethylcellulose (Pharmacoat® 603, Fa. Shin-etsu) als Bindemittel, wobei die Konzentration des Bindemittels in der Lösung aus Viskositätsgründen auf 20 Gew.-% reduziert werden mußte.

| | | |
|---|---|---|
| Eigenschaften | 10 KN Preßkraft | 18 KN Preßkraft |
| Bruchfestigkeit | 16 N | 40 N |
| Friabilität | 8,0 % | 0,6 % |
| Zerfall | 35 s. | 58 s. |

### Beispiel 10

### Verwendung als Bindemittel in einer Hydrochlorothiazid-Tablette

Eine Mischung aus 8950 g feiner Lactose (Fa. Meggle), 350 g Hydrochlorothiazid (Fa. Chemag) und 350 g Kollidon® CL (Fa. BASF) wird in einem Wirbelschichtgranulationsgerät WSG 15 (Fa. Glatt) mit einer Bindemittelzubereitung bestehend aus 350 g eines Pfropfpolymers aus Polyglycerin 2200/VAc (Beispiel 4) und 3000 g Wasser besprüht und so in der Wirbelschicht granuliert.

Folgende Prozeßparameter wurden eingestellt:

| | |
|---|---|
| Zulufttemperatur: | 90°C |
| Ablufttemperatur | 37°C |
| Sprührate | 143 g/min |
| Sprühdruck | 4 bar |

Nach der Granulation wurde noch 2,5 min bei 90°C im Gerät nachgetrocknet. Das Granulat wurde über ein 0,8 mm-Sieb gegeben, mit 5 g Magnesiumstearat (Fa. Bärlocher) versetzt und 5 min. in einem Turbulamischer (Fa. Bachofen) gemischt. Die Tablettierung erfolgte auf einer Korsch PH 106 (Fa. Korsch) Rundläuferpresse bei 18 KN Preßkraft zu biplanen, facettierten Tabletten mit 10 mm Durchmesser und 300 mg Gesamtgewicht.

| Granulateigenschaften | |
|---|---|
| Böschungswinkel | 30° |
| Aussehen | sehr gleichmäßig, ohne nennenswerten Feinanteil |

| Tabletteneigenschaften | |
|---|---|
| Bruchfestigkeit | 186 N |
| Friabilität | < 0,1 % |
| Zerfall | 4 min. 20 s. |
| Freisetzung im künstl. Magensaft (Ph. Eur.) | 92 % nach 15 min. |

### Vergleichsbeispiel

Die Herstellung erfolgte analog Beispiel 10, jedoch mit Hydroxypropylmethylcellulose (Pharmacoat® 603, Fa. Shin-etsu) als Bindemittel.

| Granulateigenschaften | |
|---|---|
| Böschungswinkel | 33° |
| Aussehen | etwas ungleichmäßig, teilweise größere Verklumpungen |

| Tabletteneigenschaften | |
|---|---|
| Bruchfestigkeit | 175 N |
| Friabilität | 0,2 % |
| Zerfall | 5 min. 10 s. |
| Freisetzung im künstl. Magensaft (Ph. Eur.) | 82 % nach 15 min. |

### Beispiel 11

### Verwendung als Hilfsstoff zur Herstellung von Ultraschallgelen

5 g p-Hydroxybenzoesäuremethylester wurden bei 50°C in 724 g demineralisiertem Wasser gelöst. Anschließend wurden 6 g Polyacrylsäure (Carbopol® 940, Fa. Goodrich) und 15 g eines Pfropfpolymerisats aus PEG 9000/VAc (Beispiel 3) unter Rühren eingearbeitet. Nach Zugabe von 200 g demineralisiertem Wasser und 50 g 4%iger wäßriger Natronlauge wurde noch 15 min gerührt, wobei darauf geachtet wurde, daß keine Luft eingearbeitet wurde. Es entstand ein Gel mit einem sehr angenehmen Hautgefühl und guten Kontakteigenschaften.

### Beispiel 12

### Verwendung als Stabilisator in einer Ibuprofensuspension

250 g Saccharose, 20 g eines Pfropfpolymers aus Lutrol® F68/VAc (Beispiel 5) und 20 g Natriumcitrat wurden in demineralisiertem Wasser gelöst. Anschließend wurden 80 g quervernetztes Polyvinylpyrrolidon (Kollidon® CL-M, BASF AG) und 40 g Ibuprofen 50 (Knoll AG) eingerührt und mit demineralisiertem Wasser auf 1000 ml aufgefüllt. Es entstand eine niedrigviskose, homogene weiße Suspension, die über Wochen Sedimentationsstabilität, keinerlei Verklumpungen oder Kuchenbildung zeigte.

### Beispiel 13

### Verwendung als Filmbildner in einem Desinfektionsspray

150 g eines Pfropfpolymers aus PEG 6000/VAc (Beispiel 1) wurden in 375 demineralisiertem Wasser gelöst und mit 375 g Ethanol versetzt. Anschließend wurden in dieser Polymerlösung unter Rühren 100 g Polyvinylpyrrolidon-Jod (PVP-Jod 30/06, BASF Aktiengesellschaft) gelöst und die Zubereitung in Pumpsprayflaschen abgefüllt. Das Desinfektionsspray zeigte sehr gute Filmeigenschaften auf der Haut und wies keinen Jodverlust nach Lagerung unter Stressbedingungen (7 Tage bei 52°C) auf.

## Patentansprüche

1. Verwendung von wasserlöslichen bzw. wasserdispergierbaren Pfropfpolymerisaten, die erhältlich sind durch Polymerisation von
a) mindestens einem Vinylester von aliphatischen C₁-C₂₄-Carbonsäuren, in Gegenwart von
b) Polyethern der allgemeinen Formel I, 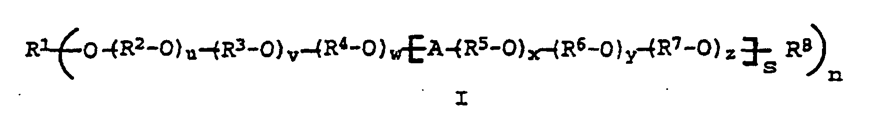
in der die Variablen unabhängig voneinander folgende Bedeutung haben:
R¹ Wasserstoff, C₁-C₂₄-Alkyl, R⁹-C(=O)-, R⁹-NH-C(=O)-, Polyalkoholrest;
R⁸ Wasserstoff, C₁-C₂₄-Alkyl, R⁹-C(=O)-, R⁹-NH-C(=O)-;
R² bis R⁷ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R⁹ C₁-C₂₄-Alkyl;
R¹⁰ Wasserstoff, C₁-C₂₄-Alkyl, R⁹-C(=O)-;
A -C(=O)-O-, -C(=O)-B-C(=O)-O-,
-C(=O)-NH-B-NH-C(=O)-O-;
B -(CH₂)ₜ-, Arylen, gegebenenfalls substituiert;
n 1 bis 8;
s 0 bis 500;
t 1 bis 12;
u 1 bis 5000;
v 0 bis 5000;
w 0 bis 5000;
x 1 bis 5000;
y 0 bis 5000;
z 0 bis 5000, **dadurch gekennzeichnet, daß** das Molekulargewicht der Polyether im Bereich von 300 bis 100000 liegt,
als Überzugsmittel, Bindemittel und/oder filmbildender Hilfsstoff in pharmazeutischen Darreichungsformen.

2. Verwendung von Polymerisaten nach Anspruch 1, die erhältlich sind durch Polymerisation von
a) mindestens einem Vinylester von aliphatischen C₁-C₂₄-Carbonsäuren, in Gegenwart von
b) Polyethern der allgemeinen Formel I,
in der die Variablen unabhängig voneinander folgende Bedeutung haben:
R¹ Wasserstoff, C₁-C₂₄-Alkyl, R⁹-C(=O)-, Polyalkoholrest;
R⁸ Wasserstoff, C₁-C₂₄-Alkyl, R⁹-C(=O)-;
R² bis R⁴ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R⁹ C₁-C₂₄-Alkyl;
R¹⁰ Wasserstoff, C₁-C₂₄-Alkyl, R⁹-C(=O)-;
n 1 bis 8;
s 0;
u 1 bis 5000;
v 0 bis 5000;
w 0 bis 5000.
als Überzugsmittel, Bindemittel und/oder filmbildender Hilfsstoff in pharmazeutischen Darreichungsformen.

3. Verwendung von Polymerisaten nach einem der Ansprüche 1 oder 2, **dadurch gekennzeichnet, daß** sie erhältlich sind durch Polymerisation von
a) mindestens einem Vinylester von aliphatischen C₁-C₁₂-Carbonsäuren, in Gegenwart von
b) Polyethern der allgemeinen Formel I mit einem mittleren Molekulargewicht von 300 bis 100.000 (nach dem Zahlenmittel), in der die Variablen unabhängig voneinander folgende Bedeutung haben:
R¹ Wasserstoff, C₁-C₁₂-Alkyl, Polyalkoholrest;
R⁸ Wasserstoff, C₁-C₁₂-Alkyl;
R² bis R⁴ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R¹⁰ Wasserstoff, C₁-C₁₂-Alkyl;
n 1 bis 5;
s 0;
u 2 bis 2000;
v 0 bis 2000;
w 0 bis 2000.

4. Verwendung von Polymerisaten nach einem der Ansprüche 1 bis 3, **dadurch gekennzeichnet, daß** sie erhältlich sind durch Polymerisation von
a) mindestens einem Vinylester von aliphatischen C₁-C₆-Carbonsäuren, in Gegenwart von
b) Polyethern der allgemeinen Formel I mit einem mittleren Molekulargewicht von 500 bis 20.000 (nach dem Zahlenmittel), in der die Variablen unabhängig voneinander folgende Bedeutung haben:
R¹, R⁸ Wasserstoff, C₁-C₆-Alkyl;
R² bis R⁴ -(CH₂)₂-, -(CH₂)₃-, -CH₂-CH(CH₃)-, -CH₂-CHOR¹⁰-CH₂-;
R¹⁰ Wasserstoff, C₁-C₆-Alkyl;
n 1;
s 0;
u 5 bis 500;
v 0 bis 500;
w 0 bis 500.

5. Verwendung von Polymerisaten nach einem der Ansprüche 1 bis 4, **dadurch gekennzeichnet, daß** zusätzlich zu den Vinylestern a) mindestens ein weiteres Monomer c), ausgewählt aus der Gruppe
c₁) C₁-C₂₄-Alkylester von monoethylenisch ungesättigten C₃-C₈-Carbonsäuren;
c₂) C₁-C₂₄-Hydroxyalkylester von monoethylenisch ungesättigten C₃-C₈-Carbonsäuren;
c₃) C₁-C₂₄-Alkyl-Vinylether;
c₄) N-Vinyllactame;
c₅) monoethylenisch ungesättigte C₃-C₈-Carbonsäuren
für die Polymerisation eingesetzt wird.

6. Verwendung von Polymerisaten nach einem der Ansprüche 1 bis 5 mit einem Verseifungsgrad von 20 bis 100 %, bezogen auf die Polyvinylestergruppen.

7. Verwendung von Polymerisaten nach einem der Ansprüche 1 bis 6 mit einem K-Wert von 10 bis 200.

8. Verwendung von Polymerisaten nach einem der Ansprüche 1 bis 7, **dadurch gekennzeichnet, daß** die Polymerisate wasserlöslich oder wasserdispergierbar sind.

9. Pharmazeutische Darreichungsformen, enthaltend mindestens einen der Polymerisate, definiert gemäß einem der Ansprüchen 1 bis 8.

10. Wasserlösliche bzw. wasserdispergierbare Pfropfpolymerisate, die erhältlich sind durch Polymerisation von
a) mindestens einem Vinylester von aliphatischen C₁-C₂₄-Carbonsäuren, in Gegenwart von
b) Polyethern der allgemeinen Formel I, 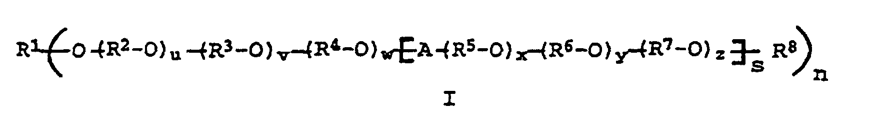
in der die Variablen unabhängig voneinander folgende Bedeutung haben:
R¹ Wasserstoff, C₁-C₂₄-Alkyl, R⁹-C(=O)-, Polyalkoholrest;
R⁸ Wasserstoff, C₁-C₂₄-Alkyl, R⁹-C(=O)-;
R² bis R⁷ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R⁹ C₁-C₂₄-Alkyl;
R¹⁰ Wasserstoff, C₁-C₂₄-Alkyl, R⁹-C(=O)-;
A -C(=O)-O-, -C(=O)-B-C(=O)-O-;
B -(CH₂)ₜ-, Arylen, gegebenenfalls substituiert;
n 1 bis 8;
s 1 bis 500;
t 1 bis 12;
u 1 bis 5000;
v 0 bis 5000;
w 0 bis 5000;
x 1 bis 5000;
y 0 bis 5000;
z 0 bis 5000, **dadurch gekennzeichnet, daß** das Molekulargewicht der Polyether im Bereich von 300 bis 100000 liegt,

11. Wasserlösliche bzw. wasserdispergierbare Pfropfpolymerisate, die erhältlich sind durch Polymerisation von
a) mindestens einem Vinylester von aliphatischen C₁-C₂₄-Carbonsäuren, in Gegenwart von
b) Polyethern der allgemeinen Formel Ia, 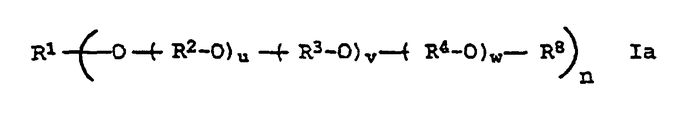 in der die Variablen unabhängig voneinander folgende Bedeutung haben:
R¹ Wasserstoff, C₁-C₂₄-Alkyl, R⁹-C(=O)-, Polyalkoholrest;
R⁸ Wasserstoff, C₁-C₂₄-Alkyl, R⁹-C(=O)-;
R² bis R⁴ -(CH₂)₂-, -(CH₂)₃-, -(CH₂)₄-, -CH₂-CH(CH₃)-,
-CH₂-CH(CH₂-CH₃)-;
R⁹ C₁-C₂₄-Alkyl;
n 1 bis 8;
u 1 bis 5000;
v 0 bis 5000;
w 0 bis 5000, **dadurch gekennzeichnet, daß** das Molekulargewicht der Polyether im Bereich von 300 bis 100000 liegt,
zusammen mit
c) mindesten einem Monomeren, ausgewählt aus der Gruppe
c₁) C₁-C₂₄-Alkylester von monoethylenisch ungesättigten C₃-C₈-Carbonsäuren;
c₂) C₁-C₂₄-Hydroxyalkylester von monoethylenisch ungesättigten C₃-C₈-Carbonsäuren;
c₃) C₁-C₂₄-Alkyl-Vinylether;
c₄) N-Vinyllactame;
c₅) monoethylenisch ungesättigte C₃-C₈-Carbonsäuren.

12. Polymerisate nach Anspruch 11, die erhältlich sind durch Polymerisation von
a) mindestens einem Vinylester von aliphatischen C₁-C₁₂-Carbonsäuren, in Gegenwart von
b) Polyethern der allgemeinen Formel Ia, in der die Variablen unabhängig voneinander folgende Bedeutung haben:
R¹ Wasserstoff, C₁-C₁₂-Alkyl, Polyalkoholrest;
R⁸ Wasserstoff, C₁-C₁₂-Alkyl;
R² bis R⁴ -(CH₂)₂-, -(CH₂)₃-, -(CH₂)₄-, -CH₂-CH(CH₃)-,
-CH₂-CH(CH₂-CH₃)-;
n 1 bis 5;
u 2 bis 2000;
v 0 bis 2000;
w 0 bis 2000
zusammen mit
c) mindestens einem Monomeren, ausgewählt aus der Gruppe
c₁) C₁-C₁₂-Alkylester von monoethylenisch ungesättigten C₃-C₈-Carbonsäuren;
c₂) C₁-C₁₂-Hydroxyalkylester von monoethylenisch ungesättigten C₃-C₈-Carbonsäuren;
c₃) C₁-C₁₂-Alkyl-Vinylether;
c₄) N-Vinyllactame;
c₅) monoethylenisch ungesättigte C₃-C₈-Carbonsäuren.

13. Polymerisate nach einem der Ansprüche 11 oder 12, die erhältlich sind durch Polymerisation von
a) mindestens einem Vinylester von aliphatischen C₁-C₆-Carbonsäuren, in Gegenwart von
b) Polyethern der allgemeinen Formel Ia, in der die Variablen unabhängig voneinander folgende Bedeutung haben:
R¹ und R⁸ Wasserstoff, C₁-C₆-Alkyl;
R² bis R⁴ -(CH₂)₂-, -(CH₂)₃-, -(CH₂)₄-, -CH₂-CH(CH₃)-,
-CH₂-CH(CH₂-CH₃)-;
n 1;
u 2 bis 500;
v 0 bis 500;
w 0 bis 500
zusammen mit
c) mindestens einem Monomeren, ausgewählt aus der Gruppe
c₁) C₁-C₆-Alkylester von monoethylenisch ungesättigten C₃-C₈-Carbonsäuren;
c₄) N-Vinylpyrrolidon, N-Vinylimidazol, N-Vinylcaprolactam;
c₅) (Meth)acrylsäure.

14. Polymerisate nach einem der Ansprüche 10 bis 13 mit einem Verseifungsgrad von 20 bis 100 %, bezogen auf die Polyvinylesterguppen.

15. Polymerisate nach einem der Ansprüche 10 bis 14 mit einem K-Wert von 10 bis 200.

16. Wasserlösliche bzw. wasserdispergierbare Pfropfpolymerisate, die erhältlich sind durch Polymerisation von
a) mindestens einem Vinylester von aliphatischen C₁-C₂₄-Carbonsäuren in Gegenwart von
b) Polyglycerin der allgemeinen Formel Ib, 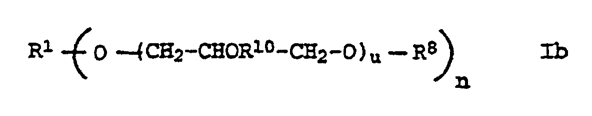
in der die Variablen unabhängig voneinander folgende Bedeutung haben:
R¹ Wasserstoff, C₁-C₂₄-Alkyl, R⁹-C(=O)-, Polyalkoholrest;
R⁸ und R¹⁰ Wasserstoff, C₁-C₂₄-Alkyl, R⁹-C(=O)-;
R⁹ C₁-C₂₄-Alkyl;
n 1 bis 8;
u 1 bis 2000, **dadurch gekennzeichnet, daß** das Molekulargewicht der Polyether im Bereich von 300 bis 100000 liegt.

17. Polymerisate nach Anspruch 16, die erhältlich sind durch Polymerisation von
a) mindestens einem Vinylester von aliphatischen C₁-C₁₂-Carbonsäuren in Gegenwart von
b) Polyglycerin der allgemeinen Formel Ib, in der die Variablen unabhängig voneinander folgende Bedeutung haben:
R¹ Wasserstoff, C₁-C₁₂-Alkyl, Polyalkoholrest;
R⁸ und R¹⁰ Wasserstoff, C₁-C₁₂-Alkyl;
n 1 bis 5;
u 1 bis 500.

18. Polymerisate nach einem der Ansprüche 16 oder 17, die erhältlich sind durch Polymerisation von
a) mindestens einem Vinylester von aliphatischen C₁-C₆-Carbonsäuren in Gegenwart von
b) Polyglycerin der allgemeinen Formel Ib, in der die Variablen unabhängig voneinander folgende Bedeutung haben:
R¹, R⁸ und R¹⁰ Wasserstoff, C₁-C₆-Alkyl;
n 1;
u 1 bis 100.

19. Polymerisate nach einem der Ansprüche 16 bis 18, **dadurch gekennzeichnet, daß** für die Polymerisation zusätzlich zu den Vinylestern mindestens ein weiteres Monomer c), ausgewählt aus der Gruppe
c₁) C₁-C₂₄-Alkylester von monoethylenisch ungesättigten C₃-C₈-Carbonsäuren;
c₂) C₁-C₂₄-Hydroxyalkylester von monoethylenisch ungesättigten C₃-C₈-Carbonsäuren;
c₃) C₁-C₂₄-Alkyl-Vinylether;
c₄) N-Vinyllactame;
c₅) monoethylenisch ungesättigte C₃-C₈-Carbonsäuren
verwendet wird.

20. Polymerisate nach einem der Ansprüche 16 bis 19, **dadurch gekennzeichnet, daß** für die Polymerisation zusätzlich zu den vinylestern mindestens ein weiteres Monomer c), ausgewählt aus der Gruppe
c₁) C₁-C₆-Alkylester von monoethylenisch ungesättigten C₃-C₈-Carbonsäuren;
c₄) N-Vinylpyrrolidon, N-Vinylimidazol, N-Vinylcaprolactam;
c₅) (Meth)acrylsäure
verwendet wird.

21. Polymerisate nach einem der Ansprüche 16 bis 20 mit einem Verseifungsgrad von 20 bis 100%, bezogen auf die Polyvinylestergruppen.

22. Polymerisate nach einem der Ansprüche 16 bis 21 mit einem K-Wert von 10 bis 200.

## Claims

1. The use of water-soluble or water-dispersible graft polymers obtainable by polymerization of
a) at least one vinyl ester of aliphatic C₁-C₂₄-carboxylic acids in the presence of
b) polyethers of the general formula I, 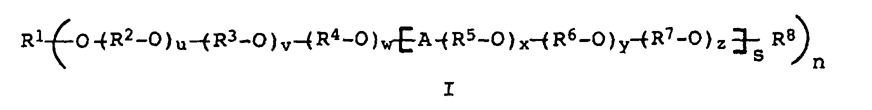
in which the variables have, independently of one another, the following meanings:
R¹ hydrogen, C₁-C₂₄-alkyl, R⁹-C(=O)-, R⁹-NH-C(=O)-, polyalcohol residue;
R⁸ hydrogen, C₁-C₂₄-alkyl, R⁹-C(=O)-, R⁹-NH-C(=O)-;
R² to R⁷ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R⁹ C₁-C₂₄-alkyl;
R¹⁰ hydrogen, C₁-C₂₄-alkyl, R⁹-C(=O)-;
A -C(=O)-O-, -C(=O)-B-C(=O)-O-,
-C(=O)-NH-B-NH-C(=O)-O-;
B -(CH₂)ₜ-, arylene, optionally substituted;
n 1 to 8;
s 0 to 500;
t 1 to 12;
u 1 to 5000;
v 0 to 5000;
w 0 to 5000;
x 1 to 5000;
y 0 to 5000;
z 0 to 5000, wherein the molecular weight of the polyethers is in the range from 300 to 100,000,
as coating agent, binder and/or film-forming excipient in pharmaceutical presentations.

2. The use of polymers as claimed in claim 1, which are obtainable by polymerization of
a) at least one vinyl ester of aliphatic C₁-C₂₄-carboxylic acids in the presence of
b) polyethers of the general formula I,
in which the variables have, independently of one another, the following meanings:
R¹ hydrogen, C₁-C₂₄-alkyl, R⁹-C(=O)-, polyalcohol residue;
R⁸ hydrogen, C₁-C₂₄-alkyl, R⁹-C(=O)-;
R² to R⁴ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R⁹ C₁-C₂₄-alkyl;
R¹⁰ hydrogen, C₁-C₂₄-alkyl, R⁹-C(=O)-;
n 1 to 8;
s 0;
u 1 to 5000;
v 0 to 5000;
w 0 to 5000,
as coating agent, binder and/or film-forming excipient in pharmaceutical presentations.

3. The use of polymers as claimed in either of claims 1 or 2, which are obtainable by polymerization of
a) at least one vinyl ester of aliphatic C₁-C₁₂-carboxylic acids in the presence of
b) polyethers of the general formula I with a number average molecular weight of from 300 to 100,000, in which the variables have, independently of one another, the following meanings:
R¹ hydrogen, C₁-C₁₂-alkyl, polyalcohol residue;
R⁸ hydrogen, C₁-C₁₂-alkyl;
R² to R⁴ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R¹⁰ hydrogen, C₁-C₁₂-alkyl;
n 1 to 5;
s 0;
u 2 to 2000;
v 0 to 2000;
w 0 to 2000.

4. The use of polymers as claimed in any of claims 1 to 3, which are obtainable by polymerization of
a) at least one vinyl ester of aliphatic C₁-C₆-carboxylic acids in the presence of
b) polyethers of the general formula I with a number average molecular weight of from 500 to 20,000, in which the variables have, independently of one another, the following meanings:
R¹, R⁸ hydrogen, C₁-C₆-alkyl;
R² to R⁴ -(CH₂)₂-, -(CH₂)₃-, -CH₂-CH(CH₃)-, -CH₂-CHOR¹⁰-CH₂-;
R¹⁰ hydrogen, C₁-C₆-alkyl;
n 1;
s 0;
u 5 to 500;
v 0 to 500;
w 0 to 500.

5. The use of polymers as claimed in any of claims 1 to 4, wherein, in addition to the vinyl esters a), at least one other monomer c) selected from the group of
c₁) C₁-C₂₄-alkyl esters of monoethylenically unsaturated C₃-C₈-carboxylic acids;
c₂) C₁-C₂₄-hydroxyalkyl esters of monoethylenically unsaturated C₃-C₈-carboxylic acids;
c₃) C₁-C₂₄-alkyl vinyl ethers;
c₄) N-vinyllactams;
c₅) monoethylenically unsaturated C₃-C₈-carboxylic acids is employed for the polymerization.

6. The use of polymers as claimed in any of claims 1 to 5 with a degree of hydrolysis of from 20 to 100% based on the polyvinyl ester groups.

7. The use of polymers as claimed in any of claims 1 to 6 with a K value of from 10 to 200.

8. The use of polymers as claimed in any of claims 1 to 7, which polymers are soluble or dispersible in water.

9. A pharmaceutical presentation comprising at least one of the polymers defined in any of claims 1 to 8.

10. A water-soluble or water-dispersible graft polymer which is obtainable by polymerization of
a) at least one vinyl ester of aliphatic C₁-C₂₄-carboxylic acids in the presence of
b) polyethers of the general formula I, 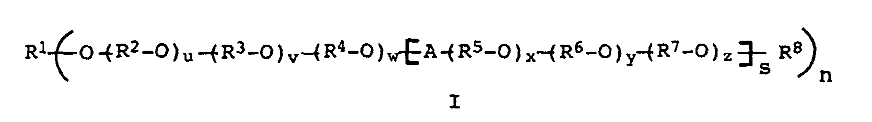
in which the variables have, independently of one another, the following meanings:
R¹ hydrogen, C₁-C₂₄-alkyl, R⁹-C(=O)-, polyalcohol residue;
R⁸ hydrogen, C₁-C₂₄-alkyl, R⁹-C(=O)-;
R² to R⁷ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R⁹ C₁-C₂₄-alkyl;
R¹⁰ hydrogen, C₁-C₂₄-alkyl, R⁹-C(=O)-;
A -C(=O)-O-, -C(=O)-B-C(=O)-O-;
B -(CH₂)ₜ-, arylene, optionally substituted;
n 1 to 8;
s 1 to 500;
t 1 to 12;
u 1 to 5000;
v 0 to 5000;
w 0 to 5000;
x 1 to 5000;
y 0 to 5000;
z 0 to 5000, wherein the molecular weight of the polyethers is in the range from 300 to 100,000.

11. A water-soluble or water-dispersible graft polymer which is obtainable by polymerization of
a) at least one vinyl ester of aliphatic C₁-C₂₄-carboxylic acids in the presence of
b) polyethers of the general formula Ia, 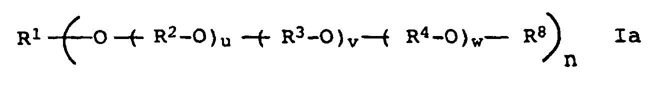 in which the variables have, independently of one another, the following meanings:
R¹ hydrogen, C₁-C₂₄-alkyl, R⁹-C(=O)-, polyalcohol residue;
R⁸ hydrogen, C₁-C₂₄-alkyl, R⁹-C(=O)-;
R² to R⁴ -(CH₂)₂-, -(CH₂)₃-, -(CH₂)₄-, -CH₂-CH(CH₃)-,
-CH₂-CH(CH₂-CH₃)-;
R⁹ C₁-C₂₄-alkyl;
n 1 to 8;
u 1 to 5000;
v 0 to 5000;
w 0 to 5000, wherein the molecular weight of the polyethers is in the range from 300 to 100,000,
together with
c) at least one monomer selected from the group of
c₁) C₁-C₂₄-alkyl esters of monoethylenically unsaturated C₃-C₈-carboxylic acids;
c₂) C₁-C₂₄-hydroxyalkyl esters of monoethylenically unsaturated C₃-C₈-carboxylic acids;
c₃) C₁-C₂₄-alkyl vinyl ethers;
c₄) N-vinyllactams;
c₅) monoethylenically unsaturated C₃-C₈-carboxylic acids.

12. A polymer as claimed in claim 11, which is obtainable by polymerization of
a) at least one vinyl ester of aliphatic C₁-C₁₂-carboxylic acids in the presence of
b) polyethers of the general formula Ia, in which the variables have, independently of one another, the following meanings:
R¹ hydrogen, C₁-C₁₂-alkyl, polyalcohol residue;
R⁸ hydrogen, C₁-C₁₂-alkyl;
R² to R⁴ -(CH₂)₂-, -(CH₂)₃-, -(CH₂)₄-, -CH₂-CH(CH₃)-,
-CH₂-CH(CH₂-CH₃)-;
n 1 to 5;
u 2 to 2000;
v 0 to 2000;
w 0 to 2000
together with
c) at least one monomer selected from the group of
c₁) C₁-C₁₂-alkyl esters of monoethylenically unsaturated C₃-C₈-carboxylic acids;
c₂) C₁-C₁₂-hydroxyalkyl esters of monoethylenically unsaturated C₃-C₈-carboxylic acids;
c₃) C₁-C₁₂-alkyl vinyl ethers;
c₄) N-vinyllactams;
c₅) monoethylenically unsaturated C₃-C₈-carboxylic acids.

13. A polymer as claimed in either of claims 11 or 12, which is obtainable by polymerization of
a) at least one vinyl ester of aliphatic C₁-C₆-carboxylic acids in the presence of
b) polyethers of the general formula Ia, in which the variables have, independently of one another, the following meanings:
R¹ and R⁸ hydrogen, C₁-C₆-alkyl;
R² to R⁴ -(CH₂)₂-, -(CH₂)₃-, -(CH₂)₄-, -CH₂-CH(CH₃)-,
-CH₂-CH(CH₂-CH₃)-;
n 1;
u 2 to 500;
v 0 to 500;
w 0 to 500
together with
c) at least one monomer selected from the group of
c₁) C₁-C₆-alkyl esters of monoethylenically unsaturated C₃-C₈-carboxylic acids;
c₄) N-vinylpyrrolidone, N-vinylimidazole, N-vinylcaprolactam;
c₅) (meth)acrylic acid.

14. A polymer as claimed in any of claims 10 to 13 with a degree of hydrolysis of from 20 to 100% based on the polyvinyl ester groups.

15. A polymer as claimed in any of claims 10 to 14 with a K value of from 10 to 200.

16. A water-soluble or water-dispersible graft polymer which is obtainable by polymerization of
a) at least one vinyl ester of aliphatic C₁-C₂₄-carboxylic acids in the presence of
b) polyglycerol of the general formula Ib, 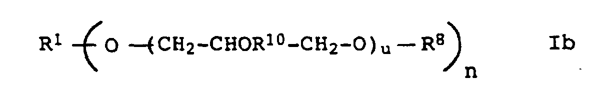
in which the variables have, independently of one another, the following meanings:
R¹ hydrogen, C₁-C₂₄-alkyl, R⁹-C(=O)-, polyalcohol residue;
R⁸ and R¹⁰ hydrogen, C₁-C₂₄-alkyl, R⁹-C(=O)-;
R⁹ C₁-C₂₄-alkyl;
n 1 to 8;
u 1 to 2000, wherein the molecular weight of the polyethers is in the range from 300 to 100,000.

17. A polymer as claimed in claim 16, which is obtainable by polymerization of
a) at least one vinyl ester of aliphatic C₁-C₁₂-carboxylic acids in the presence of
b) polyglycerol of the general formula Ib, in which the variables have, independently of one another, the following meanings:
R¹ hydrogen, C₁-C₁₂-alkyl, polyalcohol residue;
R⁸ and R¹⁰ hydrogen, C₁-C₁₂-alkyl;
n 1 to 5;
u 1 to 500.

18. A polymer as claimed in either of claims 16 or 17, which is obtainable by polymerization of
a) at least one vinyl ester of aliphatic C₁-C₆-carboxylic acids in the presence of
b) polyglycerol of the general formula Ib, in which the variables have, independently of one another, the following meanings:
R¹, R⁸ and R¹⁰ hydrogen, C₁-C₆-alkyl;
n 1;
u 1 to 100.

19. A polymer as claimed in any of claims 16 to 18, wherein, in addition to the vinyl esters, at least one other monomer c) selected from the group of
c₁) C₁-C₂₄-alkyl esters of monoethylenically unsaturated C₃-C₈-carboxylic acids;
c₂) C₁-C₂₄-hydroxyalkyl esters of monoethylenically unsaturated C₃-C₈-carboxylic acids;
c₃) C₁-C₂₄-alkyl vinyl ethers;
c₄) N-vinyllactams;
c₅) monoethylenically unsaturated C₃-C₈-carboxylic acids
is used for the polymerization.

20. A polymer as claimed in any of claims 16 to 19, wherein, in addition to the vinyl esters, at least one other monomer c) selected from the group of
c₁) C₁-C₆-alkyl esters of monoethylenically unsaturated C₃-C₈-carboxylic acids;
c₄) N-vinylpyrrolidone, N-vinylimidazole, N-vinylcaprolactam;
c₅) (meth)acrylic acid
is used for the polymerization.

21. A polymer as claimed in any of claims 16 to 20 with a degree of hydrolysis of from 20 to 100% based on the polyvinyl ester groups.

22. A polymer as claimed in any of claims 16 to 21 with a K value of from 10 to 200.

## Revendications

1. Utilisation de polymères greffés solubles dans l'eau ou dispersables dans l'eau, qui peuvent être obtenus par polymérisation de
a) au moins un ester vinylique d'acides carboxyliques aliphatiques en C₁-C₂₄, en présence de
b) des polyéthers de formule générale I, 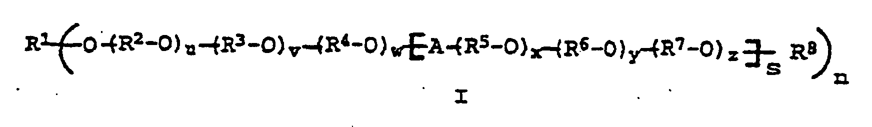
dans laquelle les variables ont, indépendamment l'une de l'autre, la signification suivante:
R¹ hydrogène, alkyle en C₁-C₂₄, R⁹-C(=O)-, R⁹-NH-C(=O)-, reste polyalcool;
R⁸ hydrogène, alkyle en C₁-C₂₄, R⁹-C(=O)-, R⁹-NH-C(=O)-;
R² à R⁷ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R⁹ alkyle en C₁-C₂₄;
R¹⁰ hydrogène, alkyle en C₁-C₂₄, R⁹-C(=O)-;
A -C(=O)-O-, -C(=O)-B-C-O-, -C(=O)-NH-B-NH-C(=O)-O-;
B -(CH₂)ₜ-, arylène, éventuellement substitué;
n 1 à 8;
s 0 à 500;
t 1 à 12;
u 1 à 5000;
v 0 à 5000;
w 0 à 5000;
x 1 à 5000;
y 0 à 5000;
z 0 à 5000, **caractérisée par le fait que** la masse moléculaire des polyéthers se situe dans l'intervalle de 300 à 100000,
comme agent de revêtement, liant et/ou adjuvant filmogène dans des formes galéniques pharmaceutiques.

2. Utilisation des polymères selon la revendication 1, qui peuvent être obtenus par polymérisation de
a) au moins un ester vinylique d'acides carboxyliques aliphatiques en C₁-C₂₄, en présence de
b) des polyéthers de formule générale I,
dans laquelle les variables ont, indépendamment l'une de l'autre, la signification suivante:
R¹ hydrogène, alkyle en C₁-C₂₄, R⁹-C(=O)-, reste polyalcool;
R⁸ hydrogène, alkyle en C₁-C₂₄, R⁹-C(=O)-;
R² à R⁴ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R⁹ alkyle C₁-C₂₄;
R¹⁰ hydrogène, alkyle en C₁-C₂₄, R⁹-C(=O)-;
n 1 à 8;
s 0;
u 1 à 5000;
v 0 à 5000;
w 0 à 5000;
comme agent de revêtement, liant et/ou adjuvant filmogène dans des formes galéniques pharmaceutiques.

3. Utilisation des polymères selon l'une des revendications 1 ou 2, **caractérisée par le fait qu'**ils peuvent être obtenus par polymérisation de
a) au moins un ester vinylique d'acides carboxyliques aliphatiques en C₁-C₁₂, en présence de
b) des polyéthers de formule générale I ayant une masse moléculaire moyenne de 300 à 100000 (selon la moyenne en nombre), où les variables ont, indépendamment l'une de l'autre, la signification suivante:
R¹ hydrogène, alkyle en C₁-C₁₂, reste polyalcool;
R⁸ hydrogène, alkyle en C₁-C₁₂;
R² à R⁴ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R¹⁰ hydrogène, alkyle en C₁-C₁₂;
n 1 à 5;
s 0;
u 2 à 2000;
v 0 à 2000;
w 0 à 2000.

4. Utilisation des polymères selon l'une des revendications 1 à 3, **caractérisée par le fait qu'**ils peuvent être obtenus par polymérisation de
a) au moins un ester vinylique d'acides carboxyliques aliphatiques en C₁-C₆, en présence de
b) des polyéthers de formule générale I ayant une masse moléculaire moyenne de 500 à 20000 (selon la moyenne en nombre), où les variables ont, indépendamment l'une de l'autre, la signification suivante:
R¹, R⁸ hydrogène, alkyle en C₁-C₆;
R² à R⁴ -(CH₂)₂-, -(CH₂)₃-, -CH₂-CH(CH₃)-,
-CH₂-CHOR¹⁰-CH₂-;
R¹⁰ hydrogène, alkyle en C₁-C₆;
n 1;
s 0;
u 5 à 500;
v 0 à 500;
w 0 à 500.

5. Utilisation de polymères selon l'une des revendications 1 à 4, **caractérisée par le fait qu'**en plus des esters vinyliques, on utilise pour la polymérisation
a) au moins un autre monomère c), choisi dans le groupe
c₁) des esters d'alkyle en C₁-C₂₄ d'acides carboxyliques en C₃-C₈ à insaturation monoéthylénique;
c₂) des esters d'hydroxyalkyle en C₁-C₂₄ d'acides carboxyliques en C₃-C₈ à insaturation monoéthylénique;
c₃) des éthers d'alkyle en C₁-C₂₄ et de vinyle;
c₄) des N-vinyllactames;
c₅) des acides carboxyliques en C₃-C₈ à insaturation monoéthylénique.

6. Utilisation de polymères selon l'une des revendications 1 à 5 ayant un degré de saponification de 20 à 100%, par rapport aux groupes poly(ester de vinyle).

7. Utilisation de polymères selon l'une des revendications 1 à 6 ayant un indice K de 10 à 200.

8. Utilisation de polymères selon l'une des revendications 1 à 7, **caractérisée par le fait que** les polymères sont solubles dans l'eau ou dispersables dans l'eau.

9. Formes galéniques pharmaceutiques, contenant au moins l'un des polymères définis selon l'une des revendications 1 à 8.

10. Polymères greffés solubles dans l'eau ou dispersables dans l'eau, qui peuvent être obtenus par polymérisation de
a) au moins un ester vinylique d'acides carboxyliques aliphatiques en C₁-C₂₄, en présence de
b) des polyéthers de formule générale I, 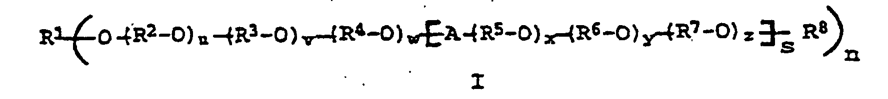
dans laquelle les variables ont indépendamment l'une de l'autre la signification suivante:
R¹ hydrogène, alkyle en C₁-C₂₄, R⁹-C(=O)-, reste polyalcool;
R⁸ hydrogène, alkyle en C₁-C₂₄, R⁹-C(=O)-;
R² à R⁷ -(CH₂)₂-, -(CH₂)₃-, -(CH₂)₄-, -CH₂-CH(CH₃)-,
-CH₂-CH(CH₂-CH₃)-, -CH₂-CHOR¹⁰-CH₂-;
R⁹ alkyle en C₁-C₂₄;
R¹⁰ hydrogène, alkyle en C₁-C₂₄, R⁹-C(=O)-;
A -C(=O)-O-, -C(=O)-B-C(=O)-O-;
B -(CH₂)ₜ-, arylène, éventuellement substitué;
n 1 à 8;
s 1 à 500;
t 1 à 12;
u 1 à 5000;
v 0 à 5000;
w 0 à 5000;
x 1 à 5000;
y 0 à 5000;
z 0 à 5000,
**caractérisés par le fait que** la masse moléculaire des polyéthers se situe dans l'intervalle de 300 à 100000.

11. Polymères greffés solubles dans l'eau ou dispersables dans l'eau, qui peuvent être obtenus par polymérisation de
a) au moins un ester vinylique d'acides carboxyliques aliphatiques en C₁-C₂₄, en présence de
b) des polyéthers de formule générale Ia, 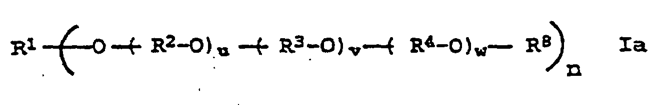 où les variables ont indépendamment l'une de l'autre la signification suivante:
R¹ hydrogène, alkyle en C₁-C₂₄, R⁹-C(=O)-, reste polyalcool;
R⁸ hydrogène, alkyle en C₁-C₂₄, R⁹-C(=O)-;
R² à R⁴ -(CH₂)₂-, -(CH₂)₃-, -(CH₂)₄-, -CH₂-CH(CH₃)-,
-CH₂-CH(CH₂-CH₃)-;
R⁹ alkyle en C₁-C₂₄;
n 1 à 8;
u 1 à 5000;
v 0 à 5000;
w 0 à 5000,
**caractérisés par le fait que** la masse moléculaire des polyéthers se situe dans l'intervalle de 300 à 100000,
conjointement avec
c) au moins un monomère, choisi dans le groupe
c₁) des esters d'alkyle en C₁-C₂₄ d'acides carboxyliques en C₃-C₈ à insaturation monoéthylénique;
c₂) des esters d'hydroxyalkyle en C₁-C₂₄ d'acides carboxyliques en C₃-C₈ à insaturation monoéthylénique;
c₃) des éthers d'alkyle en C₁-C₂₄ et de vinyle;
c₄) des N-vinyllactames;
c₅) des acides carboxyliques en C₃-C₈ à insaturation monoéthylénique.

12. Polymères selon la revendication 11, qui peuvent être obtenus par polymérisation de
a) au moins un ester vinylique d'acides carboxyliques aliphatiques en C₁-C₁₂, en présence de
b) des polyéthers de formule générale Ia, dans laquelle les variables ont indépendamment l'une de l'autre la signification suivante:
R¹ hydrogène, alkyle en C₁-C₁₂, reste polyalcool;
R⁸ hydrogène, alkyle en C₁-C₁₂;
R² à R⁴ -(CH₂)₂-, -(CH₂)₃-, -(CH₂)₄-, -CH₂-CH(CH₃)-,
-CH₂-CH(CH₂-CH₃)-;
n 1 à 5;
u 2 à 2000;
v 0 à 2000;
w 0 à 2000
conjointement avec
c) au moins un monomère, choisi dans le groupe
c₁) des esters d'alkyle en C₁-C₁₂ d'acides carboxyliques en C₃-C₈ à insaturation monoéthylénique;
c₂) des esters d'hydroxyalkyle en C₁-C₁₂ d'acides carboxyliques en C₃-C₈ à insaturation monoéthylénique;
c₃) des éthers d'alkyle en C₁-C₁₂ et de vinyle;
c₄) des N-vinyllactames;
c₅) des acides carboxyliques en C₃-C₈ à insaturation monoéthylénique.

13. Polymères selon l'une des revendications 11 ou 12, qui peuvent être obtenus par polymérisation de
a) au moins un ester vinylique d'acides carboxyliques aliphatiques en C₁-C₆, en présence de
b) des polyéthers de formule générale Ia, dans laquelle les variables ont indépendamment l'une de l'autre la signification suivante:
R¹ et R⁸ hydrogène, alkyle en C₁-C₁₂;
R² à R⁴ -(CH₂)₂-, -(CH₂)₃-, -(CH₂)₄-, -CH₂-CH(CH₃)-,
-CH₂-CH(CH₂-CH₃)-;
n 1;
u 2 à 500;
v 0 à 500;
w 0 à 500
conjointement avec
c) au moins un monomère, choisi dans le groupe
c₁) des esters d'alkyle en C₁-C₆ d'acides carboxyliques en C₃-C₈ à insaturation monoéthylénique;
c₄) des N-vinylpyrrolidone, N-vinylimidazole, N-vinylcaprolactame;
c₅) de l'acide (méth)acrylique.

14. Polymères selon l'une des revendications 1 à 13 ayant un degré de saponification de 20 à 100%, par rapport aux groupes poly(ester de vinyle).

15. Polymères selon l'une des revendications 10 à 14 ayant un indice K de 10 à 200.

16. Polymères greffés solubles dans l'eau ou dispersables dans l'eau, qui peuvent être obtenus par polymérisation de
a) au moins un ester vinylique d'acides carboxyliques aliphatiques en C₁-C₂₄, en présence de
b) de la polyglycérine de formule générale Ib, 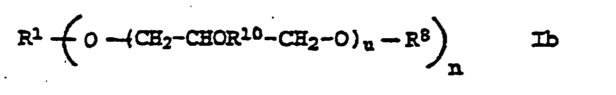
où les variables ont indépendamment l'une de l'autre la signification suivante:
R¹ hydrogène, alkyle en C₁-C₂₄, R⁹-C(=O)-, reste polyalcool;
R⁸ et R¹⁰ hydrogène, alkyle en C₁-C₂₄, R⁹-C(=O)-;
R⁹ alkyle en C₁-C₂₄;
n 1 à 8;
u 1 à 2000, **caractérisés par le fait que** la masse moléculaire des polyéthers se situe dans l'intervalle de 300 à 100000.

17. Polymères selon la revendication 16, qui peuvent être obtenus par polymérisation de
a) au moins un ester vinylique d'acides carboxyliques aliphatiques en C₁-C₁₂, en présence de
b) de la polyglycérine de formule générale Ib, dans laquelle les variables ont indépendamment l'une de l'autre la signification suivante:
R¹ hydrogène, alkyle en C₁-C₁₂, reste polyalcool;
R⁸ et R¹⁰ hydrogène, alkyle en C₁-C₁₂;
n 1 à 5;
u 1 à 500.

18. Polymères selon les revendications 16 ou 17, qui peuvent être obtenus par polymérisation de
a) au moins un ester vinylique d'acides carboxyliques aliphatiques en C₁-C₆, en présence de
b) de la polyglycérine de formule générale Ib, dans laquelle les variables ont indépendamment l'une de l'autre la signification suivante:
R¹, R8 et R¹⁰ hydrogène, alkyle en C₁-C₆;
n 1;
u 1 à 100.

19. Polymères selon l'une des revendications 16 à 18, **caractérisés par le fait que**, pour la polymérisation, on utilise en plus des esters vinyliques au moins un autre monomère c), choisi dans le groupe
c₁) des esters d'alkyle en C₁-C₂₄ d'acides carboxyliques en C₃-C₈ à insaturation monoéthylénique;
c₂) des esters d'hydroxyalkyle en C₁-C₂₄ d'acides carboxyliques en C₃-C₈ à insaturation monoéthylénique;
c₃) des éthers d'alkyle en C₁-C₂₄ et de vinyle;
c₄) des N-vinyllactames;
c₅) des acides carboxyliques en C₃-C₈ à insaturation monoéthylénique.

20. Polymères selon l'une des revendications 16 à 19, **caractérisés par le fait que**, pour la polymérisation, on utilise en plus des esters vinyliques au moins un autre monomère c), choisi dans le groupe
c₁) des esters d'alkyle en C₁-C₆ d'acides carboxyliques en C₃-C₈ à insaturation monoéthylénique;
c₄) des N-vinylpyrrolidone, N-vinylimidazole, N-vinylcaprolactame;
c₅) de l'acide (méth)acrylique.

21. Polymères selon l'une des revendications 16 à 20 ayant un degré de saponification de 20 à 100%, par rapport aux groupes poly(ester de vinyle).

22. Polymères selon l'une des revendications 16 à 21 ayant un indice K de 10 à 200.
